(19) Europäisches Patentamt
European Patent Office
Office européen des brevets

(11) EP 1 807 536 B1

(12) **FASCICULE DE BREVET EUROPEEN**

(45) Date de publication et mention de la délivrance du brevet:
**04.06.2008 Bulletin 2008/23**

(21) Numéro de dépôt: **05815224.0**

(22) Date de dépôt: **03.11.2005**

(51) Int Cl.:
***C12Q 1/68*** *(2006.01)*

(86) Numéro de dépôt international:
**PCT/FR2005/002732**

(87) Numéro de publication internationale:
**WO 2006/048553 (11.05.2006 Gazette 2006/19)**

(54) **IDENTIFICATION ET UTILISATION DE miRNAs IMPLIQUES DANS LA DIFFERENCIATION DE CELLULES ISSUES D'UNE LEUCEMIE MYELOIDE**

IDENTIFIKATION UND VERWENDUNG VON miRNAs ZUR DIFFERENZIERUNG MYELOGENER LEUKÄMIEZELLEN

IDENTIFICATION AND USE OF miRNAs FOR DIFFERENTIATING MYELOGENOUS LEUKAEMIA CELLS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **03.11.2004 FR 0411725**

(43) Date de publication de la demande:
**18.07.2007 Bulletin 2007/29**

(73) Titulaire: **CENTRE NATIONAL DE LA RECHERCHE SCIENTIFIQUE (CNRS)
75794 Paris Cedex 16 (FR)**

(72) Inventeurs:
- **VOINNET, Olivier
F-67000 Strasbourg (FR)**
- **LECELLIER, Charles-Henri
F-34920 LE CRES (FR)**
- **SAUMET, Anne
F-67000 Strasbourg (FR)**
- **LANOTTE, Michel
F-75020 Paris (FR)**

(74) Mandataire: **Bredema
38, avenue de l'Opéra
75002 Paris (FR)**

(56) Documents cités:
**WO-A-03/029459**
**WO-A-20/05078139**
**US-A1- 2005 075 492**

- **SEMPERE LORENZO F ET AL: "Expression profiling of mammalian microRNAs uncovers a subset of brain-expressed microRNAs with possible roles in murine and human neuronal differentiation." GENOME BIOLOGY 2004, vol. 5, no. 3, 16 février 2004 (2004-02-16), pages R13.1-R13.11, XP002339397 ISSN: 1465-6914**
- **KASASHIMA KATSUMI ET AL: "Altered expression profiles of microRNAs during TPA-induced differentiation of HL-60 cells." BIOCHEMICAL AND BIOPHYSICAL RESEARCH COMMUNICATIONS. 17 SEP 2004, vol. 322, no. 2, 17 septembre 2004 (2004-09-17), pages 403-410, XP002339398 ISSN: 0006-291X**
- **BENOIT GERARD ET AL: "Orchestration of multiple arrays of signal cross-talk and combinatorial interactions for maturation and cell death: Another vision of t(15;17) preleukemic blast and APL-cell maturation" ONCOGENE, vol. 20, no. 49, 29 octobre 2001 (2001-10-29), pages 7161-7177, XP002339399 ISSN: 0950-9232 cité dans la demande**
- **RUCHAUD S ET AL: "Two distinctly regulated events, priming and triggering, during retinoid-induced maturation and resistance of NB4 promyelocytic leukemia cell line" PROCEEDINGS OF THE NATIONAL ACADEMY OF SCIENCES OF THE UNITED STATES OF AMERICA, vol. 91, no. 18, 1994, pages 8428-8432, XP002339400 ISSN: 0027-8424 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- DATABASE EMBL [Online] H. sapiens C13orf25v-2 mRNA... 10 mai 2004 (2004-05-10), SETO M ET AL.: "Identification and characterization of a novel gene, C13orf25, as a target for 13q31-q32 amplification in malignant lymphoma." XP002339404 extrait de EBI Database accession no. AB176708 -& CANCER RESEARCH., vol. 64, no. 9, 1 mai 2004 (2004-05-01), pages 3087-3095, XP002339401 ISSN: 0008-5472
- CALIN GEORGE ADRIAN ET AL: "MicroRNA profiling reveals distinct signatures in B cell chronic lymphocytic leukemias" PROCEEDINGS OF THE NATIONAL ACADEMY OF SCIENCES OF THE UNITED STATES OF AMERICA, vol. 101, no. 32, 10 août 2004 (2004-08-10), pages 11755-11760, XP002339402 ISSN: 0027-8424
- OHBA HIDEKI ET AL: "Inhibition of bcr-abl and/or c-abl gene expression by small interfering, double-stranded RNAs: cross-talk with cell proliferation factors and other oncogenes." CANCER. 15 SEP 2004, vol. 101, no. 6, 15 septembre 2004 (2004-09-15), pages 1390-1403, XP002365112 ISSN: 0008-543X
- LU JUN ET AL: "MicroRNA expression profiles classify human cancers." NATURE. 9 JUN 2005, vol. 435, no. 7043, 9 juin 2005 (2005-06-09), pages 834-838, XP002339403 ISSN: 1476-4687

## Description

La présente invention est relative à un procédé pour identifier des agents thérapeutiques pour le traitement de la leucémie myéloïde, à un procédé pour identifier des miRNAs impliqués dans la différenciation de cellules leucémiques myéloïdes et à l'utilisation de miRNAs ou de séquences complémentaires à des miRNAs pour fabriquer un médicament destiné au traitement de la leucémie myéloïde.

Le terme « RNA silencing » est relatif à des mécanismes de répression de l'expression d'un gène médiés par un ARN et utilisant des interactions séquence spécifique. Chez les plantes et les animaux, il existe notamment deux voies distinctes de régulation post-transcriptionnelle de l'expression génique qui utilisent deux types différents de petits ARNs.

D'une part, on trouve les siRNAs (short interfering RNA) qui sont de petits ARNs double brin (ARNdb) de 21 à 26 nucléotides (nt) de longueur et qui agissent comme des médiateurs séquence spécifique pour la dégradation d'ARNm au cours du mécanisme d'ARN interférence (RNAi). Chez la Drosophile, ces siRNAs sont dérivés d'ARNdb par l'action d'une enzyme de type RNAse III appelée DICER. Les siRNAs formés vont alors s'associer au complexe protéique RISC (RNA-Induced Silencing Complex) qui possède une activité endonucléase. Le complexe RISC/siRNA formé va alors pouvoir couper de façon spécifique les molécules d'ARN cytoplasmique qui présentent une identité de séquence avec le siRNA présent dans le complexe. Chez les plantes et les animaux, ce mécanisme joue un rôle défensif important. Ce mécanisme, en réprimant la prolifération des éléments transposables, est également impliqué dans le maintien de l'intégrité du génome.

D'autre part, on trouve également les miRNAs (micro RNAs) qui sont de petits ARNs simple brin extrêmement conservés au cours de l'évolution et qui ont une longueur d'environ 20 nucléotides. Les miRNAs sont générés comme les siRNAs, à savoir à partir d'un précurseur double brin maturé par l'enzyme DICER. Un même précurseur ARN code pour plusieurs miRNAs. Ainsi, les miRNAs miR-19b, miR-92, miR-17, miR-18, miR-19a, miR-19b, miR-20 et miR-91 sont codés par le même précurseur ARN. De même, les miRNAs miR-23 miR-24 et miR-27 sont également codés par un même précurseur ARN. Toutefois, les miRNAs présentent néanmoins un certain nombre de différences avec les siRNAs. Ainsi, les miRNAs sont des molécules simple brin alors que les siRNAs sont des molécules doubles brins. Bien que le miRNA miR-196 soit capable de cliver l'ARNm Hox8 (YEKTA et al., Science, vol.304, p :594-596, 2004 ; MANSFIELD, Nat Genet., vol.36(10), p :1079-83, 2004), la majeure partie des miRNAs animaux n'induisent pas de clivage endonucléolytique des ARN ciblés. Au contraire, les miRNAs animaux inhibent en général la traduction des ARN ciblés en s'hybridant à leur séquence 3'UTR (région non traduite) *via* un mécanisme encore inconnu (pour une revue, voir BARTEL D.P., Cell, vol.116, p : 281-297, 2004). Pour autant les miRNAs animaux, à la différence des miRNAs végétaux présentent une homologie de séquence partielle avec leurs cibles qui pourrait justifier des différences de leur mode d'action. Enfin, à la différence des siRNAs, les miRNAs ne semblent pas impliqués dans des mécanismes de défense mais plutôt de développement, et plus particulièrement de différenciation. En effet, l'expression d'un miRNA spécifique (miR-181) dans des cellules souches hématopoïétiques en culture et *in vivo* augmente la fraction des lymphocytes B, ce qui suggère une implication de ce miRNA dans la différenciation des cellules hématopoïétiques en lymphocyte B (CHEN et al., Science, vol.303 (5654p, p :83-6). Une indication indirecte de l'importance des miRNAs dans les processus de développement chez l'animal est fournie par un arrêt de l'embryogenèse, lié à des défauts précoces du processus de différenciation, chez des souris dont le gène codant pour DICER a été muté (BERSTEIN et al., Nat. Genet., vol.35, p :215-7, 2003). À partir de ces différentes observations, il a été proposé un modèle du mécanisme de développement dans lequel pour chaque type cellulaire particulier, et à un stade de développement déterminé, un ensemble de miRNAs spécifiques influence l'expression d'une fraction déterminée du transcriptome (BARTEL D.P., 2004, précité).

Du fait du lien entre expression des miRNAs et les processus de différenciation, le profil d'expression des miRNAs au cours de la cancérogenèse suscite aujourd'hui un intérêt croissant. Il a ainsi été démontré que le miRNA let-7 est sous-exprimé dans les cancers des poumons humains et sa sur-expression dans une lignée cellulaire d'adénocarcinome de poumon inhibe la croissance cellulaire *in vitro* (TAKAMIZAWA et al., Cancer Res., vol.64, p :3753-3756, 2004).

La leucémie est qualifiée de cancer du sang et se caractérise par une prolifération des leucocytes. La leucémie peut être aiguë et entraîner la mort du patient en quelques semaines ou quelques mois. Cette maladie peut évoluer sous une forme lymphocytaire ou sous une forme myéloïde selon l'origine des cellules. La forme lymphocytaire résulte d'une hyper-prolifération des progéniteurs impliqués dans la voie de différenciation lymphoïde, alors que la forme myéloïde résulte d'une hyper-prolifération des progéniteurs impliqués dans la voie de différenciation myéloïde. Concernant plus particulièrement les leucémies myéloïdes, elles sont traitées par une combinaison de divers agents pharmacologiques qui permettent la différenciation et l'apoptose consécutive des cellules cancéreuses. Pour autant, il apparaît souvent des phénomènes de résistance au traitement, lesquels diminuent d'autant les chances de guérison du patient.

La leucémie aiguë myéloïde de type 3 (LAM3) ou encore leucémie aiguë promyélocytaire (LAP) représente près de 10% des cas de leucémie aiguë myolécytaire. Les cellules cancéreuses issues de LAM3 se caractérisent par un blocage de la granulopoïèse (voie de différenciation des granulocytes) au stade promyélocyte (DE THE et CHELBI-ALIX, oncogene, vol. 20, p :7136-9, 2001). Les cellules bloquées à un stade précoce de différenciation continuent de proliférer et s'accumulent dans la moelle osseuse. Parfois, cette accumulation de cellules s'étend à la circulation sanguine périphérique provoquant le plus souvent la mort des patients par coagulation intra-vasculaire disséminée. Au niveau moléculaire, la translocation chromosomique t(15 ;17) est associée spécifiquement à ce type de leucémie et conduit à la synthèse d'une protéine de fusion entre le récepteur $\alpha$ de l'acide rétinoïque (RAR$\alpha$) et la protéine PML. Cette protéine de fusion, baptisée PML-RAR$\alpha$ interfère négativement avec le RAR$\alpha$. Cette interférence entraîne un blocage de la différenciation des cellules au stade promyélocyte. Le traitement clinique de cette leucémie utilise des agents induisant la différenciation cellulaire (BENOIT et al., Oncogene, vol.20, p :7161-7177, 2001). Un des agents thérapeutiques anti-cancéreux les plus utilisés pour le traitement de la LAM3 est l'acide rétinoïque *tout trans* ou ATRA. L'ATRA permet la rémission de la maladie en restaurant la différenciation des cellules leucémiques et en entraînant de façon consécutive leur mort par apoptose. Cependant, comme pour les autres leucémies myéloïdes, des phénomènes de résistance sont apparus montrant les limites de l'utilisation de l'ATRA seul en thérapie anti-cancéreuse. Différentes combinaisons sont actuellement à l'étude pour développer des protocoles plus efficaces.

Par conséquent, il existe un besoin urgent d'identifier de nouvelles molécules présentant une action thérapeutique vis-à-vis des leucémies myéloïdes, de nouveaux protocoles de traitement efficaces et encore d'évaluer l'efficacité d'un traitement chez un patient atteint de leucémie myéloïde.

De façon inattendue, les inventeurs ont pu démontrer que la différenciation de cellules cancéreuses issue d'une leucémie myéloïde s'accompagne d'une modification de l'expression des miRNAs, et en particulier que la différenciation de cellules cancéreuses issues d'une leucémie myéloïde aiguë de type 3 (LAM3) s'accompagne d'une modification de l'expression des miRNAs miR23a (SEQ ID NO : 9, AUCACAUUGCCAGGGAUUUCCA), miR27a (SEQ ID NO: 11, UUCACAGUGGCUAAGUUCCGC), et miR24-2 (SEQ ID NO: 12, TGGCTCAGTTCAGCAGGAAC) codés par un même précurseur ARN (cf. figure 1) de séquence SEQ ID NO : 13 (figure 2).

Au regard de l'implication des miRNAs dans les processus de différenciation au cours de l'embryogénèse, la corrélation existant entre l'expression des miRNAs et la différenciation de cellules dérivées de leucémie myéloïde suggère que les miRNAs sont également impliqués dans le mécanisme de différenciation des cellules dérivées de leucémie myéloïde.

Les inventeurs ont pu confirmer cette implication des miRNAs dans le mécanisme de différenciation des cellules dérivées de leucémie myéloïde et mettre en évidence l'inhibition de cette différenciation en réponse à une sur-expression du précurseur ARN de séquence SEQ ID NO :13.

En conséquence, la présente invention a pour objet un procédé *in vitro* pour identifier des agents thérapeutiques ou des combinaisons d'agents thérapeutiques efficaces pour induire la différenciation de cellules leucémiques myéloïde, caractérisé en ce qu'il comprend les étapes de :

i) mise en culture de cellules issues d'une leucémie myéloïde,

ii) addition d'au moins un composé au milieu de culture de ladite lignée cellulaire,

iii) analyse de l'évolution du niveau d'expression d'au moins un miRNA codé par le précurseur ARN de séquence SEQ ID NO : 13 entre les étapes (i) et (ii),

iv) identification des composés ou de combinaisons de composés entraînant une modification du niveau d'expression dudit miRNA entre les étapes (i) et (ii).

L'étape (i) de mise en culture de cellules issues d'une leucémie myéloïde peut être effectuée selon les techniques bien connues de l'homme du métier. Des protocoles de mise en culture utilisables dans le procédé selon l'invention sont décrits notamment dans BENOIT *et al.* (2001, précité).

Selon un mode de réalisation préféré, le procédé selon l'invention permet d'identifier des agents thérapeutiques ou de combinaisons d'agents thérapeutiques efficaces pour traiter une leucémie myéloïde associée à un blocage de la granulopoïèse, et particulièrement pour traiter une leucémie myéloïde associée à un blocage au stade promyélocyte telle que la LAM3. Les cellules utilisées dans le procédé de l'invention peuvent alors être issues d'une leucémie myéloïde aiguë associée à un blocage de la granulopoïèse, et particulièrement d'une leucémie myéloïde associée à un blocage des cellules au stade promyélocyte telle que la LAM3.

Avantageusement, les cellules utilisées peuvent être des cellules de la lignée cellulaire NB4 ou une lignée cellulaire dérivée de cette dernière, une telle lignée dérivée peut être choisie parmi les lignées cellulaires NB4-LR1 et NB4-LR2 (RUCHAUD *et al.,* 1994, précité). Un protocole de mise en culture de la lignée cellulaire NB4 ou de ses lignées dérivées est notamment décrit dans BENOIT *et al.* (2001, précité).

La lignée promyélocytaire humaine NB4 a été isolée à partir d'un prélèvement de moelle osseuse d'une patiente atteinte d'une leucémie aiguë promyélocytaire (BENOIT *et al.,* 2001, précité). Ces cellules portent la translocation t(15 ;

17) et ont la capacité de se différencier en granulocytes neutrophiles sous l'effet de l'ATRA.

Les lignées NB4-LR1 et NB4-LR2, qui sont dérivées de la lignée NB4, présentent une résistance à la différenciation induite par l'ATRA. Pour la lignée NB4-LR1, si la réponse transcriptionnelle à l'ATRA est maintenue, leur différenciation nécessite un traitement conjoint ATRA/AMPc. L'étude de ce mécanisme de résistance a permis d'identifier une altération des voies de signalisation membranaires dans cette lignée qui entraîne un blocage du processus de maturation normalement enclenché par l'ATRA. Pour la lignée cellulaire NB4-LR2, ses cellules expriment une protéine PML-RARα qui est tronquée dans sa partie RARα. Cette mutation qui est localisée dans le domaine de liaison à l'acide rétinoïque de PML-RARα rend ces cellules insensibles à l'ATRA et à un mélange ATRA/AMPc. La restauration de la différenciation nécessite la coopération entre les voies de signalisation des réxinoïdes, tel que le F R 11237 ou le B M S 749 (agonistes stricts du RXR (nuclear retinoid X receptor)), et de l'AMPc. Puisque le RARα n'est plus fonctionnel, il est également possible d'utiliser l'acide rétinoïque 9-cis, agoniste à la fois du RAR et du RXR, pour induire une différenciation RXR dépendante.

Avantageusement encore, les cellules mises en culture à l'étape i) peuvent être issues d'un prélèvement, notamment d'un prélèvement sanguin, d'une personne atteinte de leucémie myéloïde. Des protocoles de mise en culture pour de telles cellules sont bien connus de l'homme du métier et sont décrits notamment dans LANOTTE et al. (Blood., vol.77, p : 1080-1086, 1991).

Selon un mode de réalisation préféré de l'invention, le ou les composés qui peuvent être utilisés à l'étape (ii) du procédé selon l'invention peuvent être de toute nature, notamment protéique, glucidique ou lipidique. L'homme du métier peut ainsi tester simplement et rapidement dans le procédé selon l'invention des composés dont il envisage qu'ils pourraient présenter un effet sur la différenciation des cellules issues d'une leucémie myéloïde.

Selon un autre mode de réalisation préféré de l'invention, le ou les composés utilisés dans l'étape (ii) du procédé selon l'invention peuvent être des agents thérapeutiques utilisés dans le traitement d'autres maladies, et plus particulièrement dans le traitement d'autres cancers. L'homme du métier peut ainsi tester simplement et rapidement dans le procédé selon l'invention des agents thérapeutiques connus dans le traitement d'autres pathologies et dont il envisage qu'ils pourraient présenter un effet sur la différenciation des cellules issues d'une leucémie myéloïde.

Selon un autre mode de réalisation préféré de l'invention, le ou les composés utilisés dans l'étape (ii) du procédé selon l'invention peuvent être des agents thérapeutiques utilisés dans le traitement de la leucémie myéloïde. Le procédé selon l'invention permet alors de déterminer les doses et/ou les combinaisons optimales d'agents thérapeutiques pour obtenir une différenciation des cellules. À titre d'exemple de tels agents thérapeutiques, on peut citer notamment l'AMPc, l'arsenic, les interférons, le TNF, l'acide rétinoïque et les dérivés des rétinoïdes, tel que l'ATRA, et les réxinoïdes.

Le composé ajouté à l'étape (ii) du procédé selon l'invention peut l'être directement au milieu de culture cellulaire à une concentration qui peut être comprise entre 1 pM et 1 M, de préférence entre 1 nM et 100 mM, et de manière particulièrement préférée entre 100 nM et 1mM.

L'étape (iii) peut être effectuée selon les techniques d'analyse connues de l'homme du métier. Par exemple, cette étape peut utiliser la technique de northern blot, de protection à la Rnase, de RT-PCR quantitative ou encore utiliser des puces à ADN intégrant des oligonucléotides complémentaires à des miRNAs. De préférence, cette étape d'analyse peut utiliser la technique de northern blot selon le protocole décrit dans LLAVE et al. (Plant Cell., vol. 14, p : 1605-1619, 2002. Pour mettre en oeuvre une telle analyse, les ARN de cellules mises en culture à l'étape (i), avant et après l'addition d'un agent thérapeutique à l'étape (ii) peuvent être extraits selon des techniques d'extraction connues de l'homme du métier. En particulier, des prélèvements de cellules peuvent être effectués de façon journalière. Les ARN purifiés peuvent ensuite être déposés sur un gel d'électrophorèse. Après migration du gel d'électrophorèse et transfert des ARN sur membrane, la membrane peut être hybridée avec une sonde marquée, froide (biotine, etc.) ou radioactive ($P^{32}$, $P^{33}$, etc.), présentant une séquence complémentaire en tout ou partie avec le précurseur ARN de séquence SEQ ID NO :13, ou à au moins un miRNA codé par ce précurseur. La séquence de la sonde présente une longueur supérieure ou égale à 10 nucléotides, de préférence à 15 nucléotides, et de manière particulièrement préférée à 20 nucléotides. La séquence de la sonde est complémentaire en tout ou partie à la séquence de l'ARN précurseur SEQ ID NO : 13, de préférence à la séquence d'au moins un miRNA codé par ce précurseur, et de manière particulièrement préférée à au moins un miRNA choisi parmi miR23a (SEQ ID NO : 9), miR27a (SEQ ID NO: 11), et miR24-2 (SEQ ID NO: 12). Après hybridation et lavage de la membrane, le signal d'hybridation correspondant au miRNA analysé peut être quantifié selon des techniques bien connues de l'homme du métier, notamment en utilisant un phosphoimager®. Le signal d'hybridation obtenu avec une sonde complémentaire à ce miRNA peut ensuite être normalisé avec le signal d'hybridation obtenu avec une sonde complémentaire à un transcrit exprimé de façon constitutive dans les cellules, tel que l'ARN 28S. La valeur normalisée obtenue pour chaque prélèvement correspond au niveau d'expression du miRNA dans les cellules pour chaque condition testée.

Selon un mode de réalisation particulier du procédé selon l'invention, les cellules utilisées à l'étape (i) peuvent être préalablement transfectées en utilisant des techniques connues de l'homme du métier par une construction contenant un gène rapporteur, tel que le gène de la GFP, et potentiellement un gène de résistance, tel qu'un gène de résistance à l'hygromycine ou à la néomycine. Le gène rapporteur contient en outre au moins une séquence complémentaire en tout ou partie à la séquence de l'ARN précurseur SEQ ID NO :13, de préférence à au moins une séquence d'un miRNA codé par ce précurseur, et de manière particulièrement préférée à au moins un miRNA choisi parmi miR23a (SEQ ID NO : 9), miR27a (SEQ ID NO: 11), et miR24-2 (SEQ ID NO: 12). Avantageusement, lesdites séquences complémentaires ont une longueur comprise entre 10 et 100 nucléotides, de préférence entre 15 et 50 nucléotides, et de manière particulièrement préférée entre 18 et 25 nucléotides. Les protocoles permettant l'obtention d'une telle construction sont connus de l'homme du métier ; un tel protocole est notamment décrit pour les siRNAs dans MANSFIELD *et al.* (2004, précité). L'utilisation d'une telle construction permet de simplifier considérablement l'analyse du niveau d'expression des différents miARNs puisqu'elle ne nécessite pas l'extraction des ARN. En effet, il a été démontré qu'un miRNA animal est capable d'induire le clivage d'un ARN lorsque ce dernier présente une séquence parfaitement complémentaire au miRNA. L'expression du gène rapporteur, notamment la GFP, est alors conditionnée à l'expression du miRNA dont une séquence complémentaire est présente au sein de la séquence codant pour le gène rapporteur. Ainsi, selon que l'agent thérapeutique induise une diminution ou une augmentation du précurseur ARN ou d'au moins un miRNA codé par celui-ci, on observera une augmentation ou une diminution de l'expression du gène rapporteur respectivement. Le suivi de l'expression du gène rapporteur pourra se faire selon des techniques connues de l'homme du métier et notamment, dans le cas de la GFP, en suivant l'émission de fluorescence des cellules transfectées.

L'étape (iv) consiste en l'identification des composés ou des combinaisons de composés entraînant une augmentation et/ou une diminution du niveau d'expression de l'ARN précurseur SEQ ID NO :13, ou d'au moins un miRNA codé par ce précurseur, de préférence d'un miRNA choisi parmi miR23a (SEQ ID NO : 9), miR27a (SEQ ID NO: 11), et miR24-2 (SEQ ID NO: 12).

Selon un mode de réalisation particulier de l'invention, l'étape (iv) consiste en l'identification des composés ou des combinaisons de composés entraînant une diminution du niveau d'expression d'au moins un desdits miRNAs. Dans ce mode de réalisation, la diminution du niveau d'expression d'au moins un desdits miRNAs peut apparaître entre le jour suivant l'addition de l'agent thérapeutique (J1) et le quatrième jour de traitement (J4).

Un deuxième procédé décrit ci-dessous est relatif à un procédé *in vitro* pour identifier des miRNAs associés à la différenciation de cellules dérivées d'une leucémie myéloïde, caractérisé en ce qu'il comprend les étapes de :

i) mise en culture d'une lignée cellulaire issue d'une leucémie myéloïde,
ii) addition, dans le milieu de culture, d'au moins un composé induisant la différenciation de ladite lignée cellulaire,
iii) analyse de l'évolution du niveau d'expression d'au moins un miRNA, ou d'un précurseur de miRNAs entre les étapes (i) et (ii),
iv) identification des miRNAs qui présentent une variation de leur profil d'expression au cours de la différenciation.

L'étape de mise en culture (i) de cellules issues d'une leucémie myéloïde peut être effectuée comme décrit précédemment.

Le procédé décrit permet d'identifier des miRNAs dont l'expression est associée à la différenciation de cellules dérivées d'une leucémie myéloïde associée à un blocage de la granulopoïèse desdites cellules, de préférence associée à un blocage au stade promyélocyte telle que la LAM3.

Avantageusement, la lignée cellulaire issue d'une leucémie myéloïde associée à un blocage de la granulopoïèse peut être la lignée cellulaire NB4 ou des lignées dérivées de celle-ci, notamment les lignées NB4-LR1 et NB4-LR2 décrites précédemment. La mise en culture desdites lignées cellulaires peut s'effectuer comme décrit dans BENOIT *et al*. (2001, précité).

L'étape (ii) d'addition de composés induisant la différenciation peut utiliser des agents thérapeutiques qui sont utilisés actuellement dans le traitement du cancer, et de préférence dans le traitement de la leucémie myéloïde. A titre d'exemple d'agents thérapeutiques utilisables dans l'étape (ii) du procédé selon l'invention, on peut citer notamment l'AMPc, l'arsenic, les interférons, le TNF, l'acide rétinoïque et les dérivés des rétinoïdes, tel que l'ATRA, les réxinoïdes. L'agent thérapeutique utilisé peut être ajouté directement au milieu de culture cellulaire à une concentration comprise entre 1 pM et 1 M, de préférence entre 1 nM et 100 mM, et de manière particulièrement préférée entre 100 nM et 1mM.

Les composés inducteurs de la différenciation pour la lignée NB4 peuvent être choisis parmi l'ATRA et un mélange ATRA/AMPc. Pour obtenir une différenciation desdites cellules, la concentration d'ATRA utilisée peut être comprise entre 1 nM et 1 mM, de préférence entre 10 nM et 100 μM, de manière particulièrement préférée entre 100 nM et 10 μM. L'ATRA peut aussi être utilisé en combinaison avec de l'AMPc présent à une concentration comprise entre 100 nM et 100 mM, de préférence entre 1 μM et 10 mM, de manière particulièrement préférée entre 10 μM et 1 mM.

Selon un second mode de réalisation préféré de l'invention, un composé inducteur de la différenciation pour la lignée NB4-LR1 peut être un mélange ATRA/AMPc. Les concentrations préférées pour ces agents thérapeutiques sont les mêmes que celles décrites précédemment.

Un composé inducteur de la différenciation pour la lignée NB4-LR2 peut être un mélange AMPc/réxinoïdes, tel qu'un mélange AMPc/F R 11237 ou AMPc/B M S 749, ou un mélange AMPc/acide rétinoïque 9-cis. Pour obtenir une différenciation desdites cellules, la concentration de réxinoïdes, tel que le F R 11237 ou AMPc/B M S 749, ou d'acide rétinoïque 9-cis peut être comprise entre 1 nM et 1 mM, de préférence entre 10 nM et 100 μM, de manière particulièrement préférée entre 100 nM et 10 μM. Les concentrations préférées pour l'AMPc sont les mêmes que celles décrites précédemment.

L'étape (iii) d'analyse peut être effectuée comme décrit précédemment, mais en utilisant comme sonde des séquences complémentaires à la séquence d'au moins un miRNA. Des séquences de miRNAs sont notamment décrites dans la demande WO 03/029459 ou sur le site internet http://www.sanger.ac.uk/Software/Rfam/mirna/index.shtml.

À titre de contrôle interne, on pourra utiliser une sonde complémentaire en tout ou partie à la séquence de l'ARN précurseur SEQ ID NO :7 (voir figure 3), de préférence à au moins un miRNA codé par ce précurseur, et de manière particulièrement préférée à au moins un miRNA choisi parmi miR-17 (SEQ ID NO :1), miR-18 (SEQ ID NO : 2), miR-19a (SEQ ID NO :3), miR-19b (SEQ ID NO :4), miR-20 (SEQ ID NO :5), miR-91 (SEQ ID NO :8) et miR-92 (SEQ ID NO :6).

L'étape (iv) consiste en l'identification des miRNAs qui présentent une variation de leur profil d'expression au cours de la différenciation de la lignée cellulaire utilisée.

Selon un mode de réalisation préféré, l'étape (iv) consiste en l'identification des miRNAs qui présentent un profil d'expression identique ou similaire à au moins un miRNA codé par l'ARN précurseur SEQ ID NO :7, de préférence à au moins un miRNA choisi parmi miR-17 (SEQ ID NO :1), miR-18 (SEQ ID NO :2), miR-19a (SEQ ID NO :3), miR-19b (SEQ ID NO :4), miR-20 (SEQ ID NO :5), miR-91 (SEQ ID NO :8) et miR-92 (SEQ ID NO :6).

Par miRNA présentant un profil d'expression identique à celui d'au moins un miRNA codé par l'ARN précurseur SEQ ID NO :7, on entend un miRNA dont les variations de niveau d'expression suivent la même cinétique et avec la même amplitude que celles d'au moins un miRNA codé par l'ARN précurseur SEQ ID NO :7 au cours de la différenciation des cellules de la lignée cellulaire utilisée, et notamment des cellules de la lignée cellulaire NB4 ou d'une lignée cellulaire dérivée de celle-ci.

Par miRNA présentant un profil d'expression similaire à celui d'au moins un miRNA codé par l'ARN précurseur SEQ ID NO :7, on entend un miRNA dont les variations de niveau d'expression suivent une cinétique décalée de quelques jours, typiquement d'un jour ou deux, et/ou avec une amplitude supérieure ou inférieure aux variations de niveau d'expression d'au moins un miRNA codé par l'ARN précurseur SEQ ID N0 : 7 au cours de la différenciation des cellules de la lignée cellulaire NB4 ou d'une lignée cellulaire dérivée.

Selon un mode de réalisation préféré du procédé décrit, les miRNAs identifiés présentent une augmentation de leur niveau d'expression en réponse à l'addition d'un agent thérapeutique inducteur de la différenciation, tel que l'ATRA ou un mélange ATRA/AMPc, entre le jour du traitement (J0) et le quatrième jour de traitement (J4), de préférence entre le premier et le troisième jour de traitement avec ledit agent thérapeutique.

Les miRNAs identifiés présentent une diminution de leur niveau d'expression en réponse à l'addition d'un agent thérapeutique inducteur de la différenciation, tel que l'ATRA, entre le deuxième (J2) et le quatrième jour de traitement (J4) de traitement avec ledit agent thérapeutique.

Un autre objet de la présente invention est relatif à l'utilisation, pour fabriquer un médicament destiné au traitement de la leucémie myéloïde, d'une molécule d'acides nucléiques choisie parmi l'ARN précurseur miR23a/24-2 (SEQ ID NO :13), une séquence dérivée d'un tel ARN, une séquence complémentaire d'un tel ARN et une séquence dérivée d'une telle séquence complémentaire.

Avantageusement, ledit médicament est une molécule d'acides nucléiques choisie parmi une séquence complémentaire de l'ARN précurseur miR23a/24-2 (SEQ ID NO :13) et une séquence dérivée d'une telle séquence complémentaire.

Selon un autre mode de réalisation préféré de l'invention, l'invention a pour objet l'utilisation, pour fabriquer un médicament destiné au traitement de là leucémie myéloïde, d'au moins une molécule d'acides nucléiques présentant une séquence choisie parmi :

i) la séquence de miR23a (SEQ ID NO :9), une séquence dérivée de miR23a, la séquence complémentaire de miR23a, une séquence dérivée d'une telle séquence complémentaire,
ii) la séquence de miR27a (SEQ ID NO :11), une séquence dérivée de miR27a, la séquence complémentaire de miR27a, une séquence dérivée d'une telle séquence complémentaire,
iii)la séquence de miR24-2 (SEQ ID NO :12), une séquence dérivée de miR24-2, la séquence complémentaire de miR24-2 et une séquence dérivée d'une telle séquence complémentaire.

Avantageusement, ledit médicament comprend une molécule d'acides nucléiques choisie parmi une séquence complémentaire de miR23a (SEQ ID NO :9), de miR27a (SEQ ID NO :11) et de miR24-2 (SEQ ID NO :12), et les séquences dérivées de telles séquences complémentaires.

De préférence, l'invention a pour objet l'utilisation d'au moins une desdites molécules d'acides nucléiques, pour fabriquer un médicament destiné au traitement d'une leucémie myéloïde associée à un blocage de la granulopoïèse, et de manière particulièrement préférée à un blocage au stade promyélocytes, telle que la LAM3.

Les molécules d'acides nucléiques peuvent être utilisées sous forme simple brin ou double brin, de préférence sous forme simple brin. Les acides nucléiques peuvent être sélectionnés parmi l'ADN, l'ARN ou les acides nucléiques modifiés tels que les ribonucléotides ou les désoxyribonucléotides présentant un groupement sucre ou un groupement carboné modifié.

Les molécules d'ARN ou d'ADN utilisées dans la présente invention peuvent également contenir un ou plusieurs nucléotides modifiés, c'est-à-dire un ribonucléotide ou désoxyribonuléotide naturel substitué par un analogue synthétique d'un nucléotide. De tels analogues de nucléotides peuvent par exemple être localisés à l'extrémité 3' ou 5' de la molécule d'acide nucléique.

Des analogues synthétiques de nucléotides préférés sont sélectionnés parmi les ribonucléotides présentant un groupement sucre ou groupement carboné modifié. De préférence, les ribonucléotides présentant un groupement sucre modifié présentent un groupement 2'-OH remplacé par un groupement sélectionné parmi un atome d'hydrogène, un halogène, un groupement OR, R, SH, SR, $NH_2$, NHR, $NR_2$ ou CN, dans lequel R est un groupement alkyle, alcényle ou alcynyle de 1 à 6 carbone et l'halogène est le fluor, le chlore, le brome ou l'iode. De préférence, les ribonucléotides présentant un groupement carboné modifié ont leur groupement phosphoester lié au ribonucléotide adjacent qui est remplacé par un groupement modifié tel qu'un groupement phosphthioate. Pour autant, il est également possible d'utiliser des ribonucléotides présentant un noyau purine ou pyrimidine modifié. Comme exemples de tels noyaux modifiés, on citera notamment les uridines ou les cytidines modifiées en position 5, telles que la 5-(2-amino)propyl uridine et la 5-bromo uridine, les adénosines et guanosines modifiées en position 8, telle que la 8-bromo guanosine, les nucléotides déazotés, telle que la 7-déaza-adénosine, les nucléotides N- et O-alkylés, telle que la N6-méthyl adénosine. Ces différentes modifications peuvent également être combinées.

Les molécules d'acide nucléique utilisées dans la présente invention peuvent être obtenues par des méthodes de synthèse chimique ou par des méthodes de biologie moléculaire, notamment par transcription à partir de matrices ADN ou de plasmides isolés à partir de microorganismes recombinants. De préférence, cette étape de transcription utilise des ARN polymérase de phage telles que l'ARN polymérase T7, T3 ou SP6.

Par séquence dérivée, on entend une séquence présentant une identité d'au moins 80%, de préférence d'au moins 90%, et de manière particulièrement préférée d'au moins 95% avec une séquence de référence. La détermination d'une identité de séquence est effectuée selon la formule suivante :

$$I = n / L$$

Où I représente l'identité en pourcentage (%), n est le nombre de nucléotides identiques entre une séquence donnée et une séquence de miRNA donnée et L est la longueur de la séquence. Les nucléotides A, C, G et U peuvent correspondre à des ribonucléotides, des déoxyribonucléotides et/ou à des analogues de nucléotides, comme des analogues synthétiques de nucléotide. En outre, les nucléotides peuvent être substitués par des nucléotides formant des liaisons hydrogènes analogues avec une séquence nucléique complémentaire. Ainsi, le nucléotide U peut être substitué par un nucléotide T.

Les molécules d'acides nucléiques utilisées pour fabriquer un médicament destinées au traitement de la leucémie myéloïde ont de préférence une longueur comprise entre 15 et 100 nucléotides, préférentiellement entre 18 et 80 nucléotides et de manière particulièrement préférée entre 18 et 30 nucléotides. Pour les molécules de miRNA matures, elles présentent une longueur comprise entre 19 et 24 nucléotides, et plus particulièrement de 21, 22 ou 23 nucléotides. Avantageusement, la séquence complémentaire à un miRNA présente une longueur comprise entre 19 et 24 nucléotides. Pour autant, il est possible d'utiliser la séquence dérivée d'un précurseur de miRNAs d'une longueur comprise entre 50 et 90 nucléotides, le plus souvent entre 60 et 80 nucléotides, mais qui peut aussi présenter une longueur supérieure à 100 nucléotides.

L'administration des molécules d'acides nucléiques peut être effectuée par des méthodes de transfert de gènes connues dans l'homme du métier.

Des méthodes communes de transfert de gène incluent le phosphate de calcium, le DEAE-Dextran, l'électroporation, la microinjection, les méthodes virales et les liposomes cationiques (GRAHAM et VAN DER EB, Virol., vol.52, p :456, 1973 ; McCUTHAN et PAGANO, J. Natl. Cancer Inst., vol.41, p :351, 1968 ; CHU et al., Nucl. Acids Res., vol. 15, p :1311 ; FRALEY et al., J. Biol. Chem., vol.255, p :10431, 1980 ; CAPECCHI et al., Cell, vol. 22, p :479, 1980 ; FELGNER iet al., Proc. Natl. Acad. Sci. USA, vol.84, p :7413, 1988).

Les molécules d'acides nucléiques à administrer peuvent être sous la forme d'une solution, notamment d'une solution injectable, d'une crème, d'un comprimé ou encore d'une suspension. Le support peut être tout support pharmaceutique. De préférence, on utilisera un support capable d'améliorer l'entrée des molécules d'acides nucléiques dans les cellules. De tels supports incluent notamment les liposomes, de préférence les liposomes cationiques.

Une quantité efficace de molécules d'acides nucléiques à administrer à un patient peut être déterminée simplement par l'homme du métier. À titre d'exemple, une quantité efficace de molécules d'acides nucléiques est comprise entre 0,001mg et 10g/kg du patient à traiter, de préférence entre 0,01mg et 1g/kg, et de manière particulièrement préférée entre 0,1 et 100mg/kg.

Les exemples qui suivent sont fournis à titre d'illustration et ne sauraient limiter la portée de la présente invention.

**Exemple 1 Différenciation des cellules NB4 et NB4-LR1-en présence d'ATRA et/ou d'AMPc**

Les cellules de la lignée cellulaire NB4 et de la lignée cellulaire NB4-LR1, résistante à la maturation par l'ATRA seul, ont été cultivées comme décrit dans LANOTTE *et al.* (1991, précité) et dans RUCHAUD et al. (Proc. Natl. Acad. Sci., vol. 91, p :8428-8432, 1994). Les cellules ont ensuite été traitées pendant 4 jours en présence de 1μM d'acide rétinoïque *tout-trans* (ATRA, SIGMA-ALRICH) seul ou additionné de 100 μM d'un analogue d'AMPc (8-CPT-cAMP, SIGMA-ALRICH).

La prolifération cellulaire a été déterminée journellement par comptage des cellules en utilisant un compteur de cellules (BECKMAN COULTER FRANCE SA) du jour 0, suivant l'addition de l'agent thérapeutique, au jour 4. Les résultats montrent que les différents traitements induisent une diminution de la prolifération.

Parallèlement, l'évolution de la différenciation a été déterminée journellement au cours du traitement. La différenciation granulocytique a été évaluée simultanément selon des critères morphologiques et biochimiques. L'analyse morphologique a été effectuée après une coloration de May-Grünwald. L'analyse biochimique a été effectuée par un test de coloration basé sur la réduction du NBT (nitro blue tetrazolium) qui permet de mesurer la capacité oxydative des cellules matures à réduire le colorant NBT. À cet effet, 0,5 à 1 x $10^5$ cellules ont été centrifugées pendant 5 min à 190 g. le culot cellulaire a ensuite été repris dans 200 μl de tampon salin phosphate (PBS) additionné de NBT (SIGMA ALDRICH, 1 mg/ml) et de PMA (Phorbol 12-myristate 13-acetate, SIGMA, $10^{-7}$M), puis a été incubé 20 minutes à 37°C. Les cellules ont ensuite été récoltées sur lames par centrifugation Cytospin®, puis observées par microscopie par contraste de phase. Un minimum de 200 cellules par lame a été examiné sous microscope optique et un pourcentage de différenciation a été calculé sur la base du nombre de cellules NBT positives. Les résultats obtenus sont résumés dans le tableau I ci-dessous :

Tableau I

| Lignée cellulaire | Traitement | Évolution du pourcentage de cellules différenciées au cours du traitement (en jours) | | | | |
|---|---|---|---|---|---|---|
| | | 0 | 1 | 2 | 3 | 4 |
| NB4 | ATRA | 0% | 6% | 20% | 43% | 95% |
| | ATRA+AMPc | 0% | 6% | 30% | 80% | 100% |
| NB4-LR1 | ATRA | 0% | 0% | 2% | 5% | 5% |
| | ATRA+AMPc | 0% | 5% | 25% | 75% | 95% |

Les résultats montrent que le co-traitement ATRA/AMPc permet d'obtenir une différenciation des cellules NB4 et NB4-LR1. En revanche, seules les cellules de la lignée cellulaire NB4 se différencient en présence de l'ATRA seul. La figure 4 montre un exemple de coloration de cellules NB4 au NBT avant et après trois jours de traitement à l'ATRA. On observe alors nettement le changement de morphologie des cellules résultant de leur différenciation.

**Exemple 2 : Expression des miRNAs au cours de la différenciation des cellules NB4 induite par l'ATRA**

Dans une première série d'expérience, l'expression de différents miRNAs a été évaluée au cours de la différenciation des cellules NB4 en présence ou en l'absence d'ATRA. L'expression des miRNAs suivants a notamment été déterminée :

| | | |
|---|---|---|
| miR23a | (SEQ ID NO : 9) | AUCACAUUGCCAGGGAUUUCCA |
| miR27a | (SEQ ID NO: 11) | UUCACAGUGGCUAAGUUCCGC |
| miR24-2 | (SEQ ID NO: 12) | UGGCUCAGUUCAGCAGGAACAG |
| miR15a | (SEQ ID NO: 14) | UAGCAGCACAUAAUGGUUUGUG |
| miR16 | (SEQ ID NO: 15) | UAGCAGCACGUAAAUAUUGGCG |
| miR19b | (SEQ ID NO : 4) | UGUGCAAAUCCAUGCAAAACUGA |
| miR92 | (SEQ ID NO : 6) | UAUUGCACUUGUCCCGGCCUGU |
| miR19a | (SEQ ID NO : 3) | UGUGCAAAUCUAUGCAAAACUGA |

(suite)

| | | |
|---|---|---|
| miR20 | (SEQ ID NO : 5) | UAAAGUGCUUAUAGUGCAGGUA |
| miR17 | (SEQ ID NO : 1) | CAAAGUGCUUACAGUGCAGGUAGU |
| miR18 | (SEQ ID NO : 2) | UAAGGUGCAUCUAGUGCAGAUA |
| miR91 | (SEQ ID NO : 8) | ACUGCAGUGAAGGCACUUGU |
| let-7a | (SEQ ID NO: 17) | UGAGGUAGUAGGUUGUAUAGUU |
| let-7d | (SEQ ID NO: 18) | AGAGGUAGUAGGUUGCAUAGU |
| miR15b | (SEQ ID NO: 19) | UAGCAGCACAUCAUGGUUUACA |
| miR142S | (SEQ ID NO: 20) | CAUAAAGUAGAAAGCACUAC |
| miR223 | (SEQ ID NO: 21) | UGUCAGUUUGUCAAAUACCCC |
| miR320 | (SEQ ID NO: 22) | AAAAGCUGGGUUGAGAGGGCGAA |
| miR422b | (SEQ ID NO: 23) | CUGGACUUGGAGUCAGAAGGCC |

Parmi ces miRNAs, certains appartiennent à un même précurseur, ainsi (A) les miRNAs miR-19b, miR-92 miR-17, miR-18, miR-19a, miR-19b, miR-20, miR-91 et miR-92, (B) les miRNAs miR15a et miR16, et (C) les miRNAs miR23a, miR27a et miR24-2 (cf. figure 1). Un modèle pose que de tels miRNAs générés à partir d'un même précurseur ARN présentent un même profil d'expression (LEE et al., Embo J., vol. 21, p : 4663-4670, 2002).

Les ARN totaux ont été extraits des cellules des lignées cellulaires NB4 traitées ou non avec 1μM d'ATRA, aux mêmes intervalles de temps qu'à l'exemple 1, et en utilisant le kit Tri-Reagent® (SIGMA) selon les instructions du fabricant. L'analyse par northern des ARN de faible poids moléculaire a été effectuée comme décrit dans LLAVE *et al.* (2002, précité). Toutes les expériences de northern ont été effectuées en double. Des oligonucléotides ADN complémentaires aux séquences des miRNAs analysés ont été marqués à leur extrémité avec de l'ATP $\gamma$-$P^{32}$ en utilisant la polynucléotide kinase T4 (NEW ENGLAND BIOLABS) en suivant les instructions du fabricant.

La figure 5 montre le profil d'expression des différents miRNAs analysés dans les cellules NB4 après 0, 1, 2, 3 et 4 jours de traitement. La quantité d'ARN dans chaque puits a été contrôlée par coloration du gel au bromure d'éthidium et visualisation des ARN ribosomiques (ARNr) sous lumière UV.

Les résultats montrent que l'induction de la différenciation des cellules NB4 en présence d'ATRA induit une modification de l'expression de nombreux miRNAs (voir figure 5). En outre, il apparaît que les miRNAs appartenant à un même précurseur présentent effectivement un profil d'expression similaire au cours du temps.

De façon plus détaillée, les résultats obtenus montrent une modulation du niveau d'expression des miRNAs miR-19b, miR-23a et miR-92 au cours de la différenciation des cellules NB4 en réponse au traitement à l'ATRA. Dans le cas de miR-23a, son niveau d'expression augmente au cours du temps en réponse au traitement à l'ATRA.

Le profil d'expression de miR-19b et de miR-92 diffère de celui de miR-23. Ce profil d'expression correspond à une première augmentation du niveau d'expression de miR-19b et miR-92, immédiatement après le traitement (J0) avec ensuite un maximum d'expression au troisième jour de traitement. Finalement, le niveau d'expression de miR-19b et de miR-92 chute entre le $3^{ème}$ et le $4^{ème}$ jour de traitement (entre J3 et J4) alors que la différenciation des granulocytes est complète (cf. tableau I). En outre, le niveau d'expression de miR-19b et de miR-92 au $4^{ème}$ jour de traitement est inférieur à leur niveau d'expression dans les cellules non traitées.

## Exemple 3 : Expression des miRNAs dans les cellules des lignées cellulaires NB4 et NB4-LR1 en réponse à un traitement à l'ATRA

Pour établir formellement la corrélation entre l'expression des différents miRNAs identifiés et la différenciation en granulocytes, des cellules des lignées cellulaires NB4 et NB4-LR1 ont été cultivées en présence ou en l'absence d'ATRA comme décrit à l'exemple 1.

Des expériences de northern ont été effectuées selon le protocole décrit à l'exemple 2. Les différentes expériences de northern ont été réalisées avec des sondes complémentaires aux miRNAs miR-23a, miR-17, miR-18, miR-19a, miR-19b, miR-20, miR-23 et miR-92.

Les résultats obtenus montrent que le profil d'expression de miR-23 est similaire dans les cellules NB4 et NB4-LR1 en réponse à un traitement à l'ATRA (voir figures 5 et 6). En revanche, on n'observe aucune augmentation de l'expression de miR23a dans le cas d'un traitement des cellules NB4-LR2 avec l'ATRA.

En revanche, les résultats ont montré que les différents miRNAs miR-17, miR-18, miR-19a, miR19b, miR-20 et miR-92, lesquels sont codés par un même précurseur ARN, présentent un profil d'expression différent entre les cellules NB4 et NB4-LR1 en réponse au traitement à l'ATRA.

**Exemple 4 : Expression des miRNA dans les cellules des lignées cellulaires NB4 et NB4-LR1 en réponse à un traitement simultané à l'ATRA et à l'AMPc**

Pour confirmer la corrélation entre l'expression des différents miRNAs, et notamment miR-17, miR-18, miR-19a, miR19b, miR-20 et miR-92 et la différenciation en granulocytes, des cellules des lignées cellulaires NB4 et NB4-LR1 ont été cultivées en présence ou en l'absence d'ATRA/AMPc comme décrit à l'exemple 1. À la suite d'un co-traitement ATRA/AMPc, la différenciation des cellules de la lignée cellulaire NB4-LR1 en granulocyte est restaurée.

Des expériences de northern ont été effectuées selon le protocole décrit à l'exemple 2. Les différentes expériences de northern ont été réalisées avec des sondes complémentaires aux miRNAs miR-17, miR-18, miR-19a, miR-19b, miR-20 et miR-92.

Les résultats ont montré que le profil d'expression de ces différents miRNAs est identique entre les cellules NB4 et NB4-LR1 en réponse au co-traitement ATRA/AMPc. Pour autant, le profil d'expression de ces miRNAs diffère de celui observé dans les cellules NB4 en réponse au traitement à l'ATRA seul. En réponse au co-traitement ATRA/AMPc, les miRNAs miR-17, miR-18, miR-19a, miR-19b, miR-20 et miR-92 montrent en effet un maximum d'expression au $2^{ème}$ jour de traitement, puis une diminution importante de leur niveau d'expression entre le $2^{ème}$ et le $3^{ème}$ jour de traitement, enfin leur niveau d'expression l'initial se rétablit entre le $3^{ème}$ et le $4^{ème}$ jour d'expression.

L'induction et la chute consécutive du niveau d'expression des miRNAs miR-17, miR-18, miR-19a, miR-19b, miR-20 et miR-92 s'opère donc de manière plus précoce en réponse au co-traitement ATRA/AMPc (par rapport au traitement à l'ATRA seul), tout comme la différenciation des cellules NB4 en granulocytes (cf. tableau I).

Les résultats obtenus confortent donc la corrélation entre l'expression de certains miRNAs, et notamment les miRNAs miR-17, miR-18, miR-19a, miR19b, miR-20 et miR-92 et la différenciation des cellules NB4 et NB4-LR1 en granulocytes. En outre, la cinétique d'expression différente des miRNAs étudiés entre le traitement à l'ATRA et le co-traitement ATRA/AMPc peut se justifier par la cinétique de différenciation différente entre les deux traitements. Enfin, la cinétique d'expression des miRNAs miR-17, miR-18, miR-19a, miR19b, miR-20 et miR-92 montre donc successivement une induction de leur expression avec l'initiation de la différenciation, puis une inhibition de leur expression en fin de différenciation.

**Exemple 5 : Différenciation des cellules NB4 en réponse à une surexpression de différents miRNAs**

Pour déterminer la possible implication de certains des miRNAs analysés dans la granulopoïèse, les séquences génomiques codant pour l'ARN précurseur de séquence SEQ ID NO : 7 codant pour les miRNAs miR17/92 (voir figure 3, la séquence complémentaire de la séquence codant pour l'ARN précurseur de séquence SEQ ID NO : 7 est représentée dans la figure 8, SEQ ID NO : 10), pour l'ARN précurseur de séquence SEQ ID NO : 13 codant pour les miRNAs miR23a/24-2 et pour l'ARN précurseur de séquence SEQ ID NO : 16 (voir figure 7) codant pour les miRNAs miR16/15a ont été clonées en amont du site d'entrée interne ribosomique (IRES) du vecteur MIE (MSCV IRES EGFP (enhanced green fluorescent protein), SYSTEMIX) comme décrit dans CHANGCHUN et al. (Blood, vol.94(2), p :793-802, 1999). La production de surnageants contenant les différents rétrovirus MIE-miRNA (MIE contenant notamment la séquence génomique SEQ ID NO : 10 sous contrôle d'un promoteur pol II) dans la lignée cellulaire Bosc 23 (PEAR et al., Proc. Natl. Acad. Sci. USA, vol.90, p8392-8396, 1993) a ensuite été effectuée comme décrit dans LAVAU et al. (EMBO J., vol.16, p :4226-4237, 1997). Des cellules de la lignée cellulaire NB4 ont ensuite été infectées par les différents rétrovirus et sélectionnées selon le protocole décrit dans CHANGCHUN *et al.* (1999, précité).

Les cellules de la lignée cellulaire NB4 infectées par le vecteur MIE seul ou par les différents vecteurs MIE-miRNA, sont ensuite cultivées comme décrit à l'exemple 1 en présence ou en l'absence d'ATRA.

L'évolution de la différenciation a été déterminée journellement pour les différentes cultures comme décrit à l'exemple 1.

Les résultats montrent qu'à la différence des cellules infectées par les vecteurs MIE seul, MIE-miR17/miR92 et miR15a/16, lesquelles se différencient au bout de quatre jours de culture en présence d'ATRA, les cellules infectées par le vecteur MIE-miR23a/miR24-2 ne se différencient pas dans les mêmes conditions (voir figures 9 et 10).

Afin de confirmer la sur-expression des miRNAs dans les cellules NB4 infectées, des expériences de northern ont été effectuées selon le protocole décrit à l'exemple 2. Les différentes expériences de northern ont été réalisées avec des sondes complémentaires aux miRNAs miR-23a, miR16 et miR-92.

Les résultats confirment que les cellules infectées- par les vecteurs MIE-miRNAs présentent un niveau d'expression pour les miRNAs codés par le vecteur MIE-miRNAs utilisé pour les infecter (voir figure 11).

En conséquence, et de façon inattendue, les résultats suggèrent que les miRNAs miR23a, miR27a et miR24-2 sont potentiellement impliqués dans la différenciation des cellules NB4 en présence d'ATRA, et plus précisément que ces miRNAs régulent « négativement » la voie de différenciation de la granulopoïèse dans les cellules NB4.

**Exemple 6: Différenciation des cellules NB4 en réponse à une surexpression de différents miRNAs codés par le précurseur miR23a/24-2**

Pour déterminer individuellement l'implication des différents miRNAs codés par le précurseur miR2a/24-2 dans la granulopoïése induite par l'ATRA, la séquence génomique codant pour l'ARN précurseur de séquence SEQ ID NO: 13 codant pour les miRNAs miR23a/24-2, ainsi que différentes constructions présentant une délétion pour un ou deux miRNAs codés par ce dernier (Δ24Δ27, Δ24, Δ23Δ24, Δ23, Δ23Δ27 et Δ27) ont été clonées en amont du site d'entrée interne ribosomique (IRES) du vecteur MIE comme décrit précédemment.

Des cellules de la lignée cellulaire NB4 ont alors été infectées par ces différents vecteurs retrovirus, puis sélectionnées selon le protocole décrit à l'exemple 5, puis cultivées comme décrit à l'exemple 1 en présence ou en l'absence d'ATRA.

L'évolution de la différenciation a été déterminée journellement pour les différentes cultures comme décrit à l'exemple 1.

Les résultats montrent que seul le vecteur intégrant le précurseur complet miR23a/24-2 permet de bloquer la différenciation des cellules NB4 en présence d'ATRA (voir figure 12). Des expériences de northern blot effectuées selon le protocole décrit précédemment montrent que les vecteurs codant pour des précurseurs tronqués miR23a/24-2 permettent pour autant d'obtenir la sur-expression des miRNAs effectivement codés par ces derniers (voir figure 13).

En conséquence, l'expression coordonnée des miRNAs miR23a, miR27a et miR24-2 est nécessaire pour obtenir un blocage de la différenciation des cellules NB4 en présence d'ATRA.

Afin de déterminer si le précurseur complet miR23a/24-2 est nécessaire à l'inhibition de la différenciation des cellules NB4 en présence d'ATRA, des cellules NB4 ont été co-infectées par le vecteur MIE seul ou par les vecteurs MIE-Δ23Δ27 et MIE-Δ24 simultanément.

Les résultats montrent alors que la complémentation en trans des différents miRNAs du précurseur miR23a/24-2 ne permet pas de bloquer la différenciation des cellules NB4 en présence d'ATRA (voir figure 14). Pour autant, les expériences de northern effectuées sur les cellules infectées selon le protocole décrit précédemment montrent que les différents miRNAs miR23a, miR27a et miR24-2 sont bien sur-exprimés dans les cellules infectées simultanément par les vecteurs MIE-Δ23Δ27 et MIE-Δ24 (voir figure 15).

En conclusion, et de façon inattendue, ces expériences montrent que l'expression des miRNAs miR23a, miR27a et miR24-2 simultanément et à partir d'un même précurseur est nécessaire pour inhiber la différenciation des cellules NB4 en présence d'ATRA.

**Exemple 7 Différenciation des cellules NB4 en réponse à une inhibition de l'expression des miRNAs miR23a, miR27a et miR24-2**

Des oligonucléotides chimiquement modifiés (LNA®-DNA, PROLIGO) et de séquence complémentaires aux miR23a, miR27a et miR24-2 ont été synthétisés. Ces oligonucléotides modifiés sont composés d'analogues de nucléotides contenant un pont 2'-O, 4'-C méthylène qui permet d'améliorer aussi bien la stabilité de l'oligonucléotide obtenu que les performances d'hybridation de celui-ci.

Des cellules de la lignées cellulaires NB4 ou NB4-LR1 ont ensuite été transfectées par les oligonucléotides synthétisés selon le protocole décrit dans MEISTER et al. (RNA, vol.10(3), p :544-50, 2004).

Les cellules des lignées cellulaires NB4 et NB4-LR1, transfectées ou non par un oligonucléotide complémentaire aux miRNAs miR23a, miR27a et miR24-2 sont ensuite cultivées comme décrit à l'exemple 1 en présence ou en l'absence d'ATRA ou d'un mélange ATRA/AMPc.

L'évolution de la différenciation a été déterminée journellement pour les différentes cultures comme décrit à l'exemple 1.

Parallèlement, des expériences de northern ont été effectuées selon le protocole décrit à l'exemple 2. Les différentes expériences de northern ont été réalisées avec des sondes complémentaires aux miRNAs miR23a, miR27a et miR24-2.

## Revendications

1. Utilisation, pour fabriquer un médicament destiné au traitement de la leucémie myéloïde, d'une molécule d'acides nucléiques ayant une longueur d'au moins 15 nucléotides choisie parmi l'ARN précurseur miR23a/24-2 (SEQ ID NO :13), une séquence dérivée présentant une identité d'au moins 80% avec un tel ARN, une séquence complémentaire d'un tel ARN et une séquence dérivée présentant une identité d'au moins 80% avec une telle séquence complémentaire.

2. Utilisation selon la revendication 1, **caractérisé en ce que** ledit médicament comprend une molécule d'acides nucléiques choisie parmi une séquence complémentaire de l'ARN précurseur miR23a/24-2 (SEQ ID NO :13) et une séquence dérivée d'une telle séquence complémentaire.

3. Utilisation selon l'une quelconque des revendications 1 ou 2, **caractérisé en ce que** ledit médicament comprend une molécule d'acides nucléiques présentant une séquence choisie parmi :

    i) la séquence de miR23a (SEQ ID NO :9), une séquence dérivée de miR23a, la séquence complémentaire de miR23a, une séquence dérivée d'une telle séquence complémentaire,
    ii) la séquence de miR27a (SEQ ID NO :11), une séquence dérivée de miR27a, la séquence complémentaire de miR27a, une séquence dérivée d'une telle séquence complémentaire,
iii) la séquence de miR24-2 (SEQ ID NO :12), une séquence dérivée de miR24-2, la séquence complémentaire de miR24-2 et une séquence dérivée d'une telle séquence complémentaire.

4. Utilisation selon la revendication 3, **caractérisé en ce que** ledit médicament comprend une molécule d'acides nucléiques choisie parmi une séquence complémentaire de miR23a (SEQ ID NO :9), de miR27a (SEQ ID NO :11) et de miR24-2 (SEQ ID NO :12), et les séquences dérivées de telles séquences complémentaires.

5. Utilisation selon l'une quelconque des revendications 1 à 4, pour fabriquer un médicament destiné au traitement d'une leucémie myéloïde associée à un blocage de la granulopoïèse.

6. Utilisation selon l'une quelconque des revendications 1 à 5, **caractérisée en ce que** les séquences d'acides nucléiques ont une longueur comprise entre 15 et 100 nucléotides.

7. Utilisation selon l'une quelconque des revendications 1 à 6, **caractérisée en ce que** les molécules d'acides nucléiques sont choisies parmi les molécules d'ADN et d'ARN.

8. Utilisation selon l'une quelconque des revendications 1 à 7, **caractérisée en ce que** les molécules d'acides nucléiques contiennent un ou plusieurs nucléotides modifiés.

9. Utilisation selon l'une quelconque des revendications 1 à 8, **caractérisée en ce que** les molécules d'acides nucléiques sont sous forme simple brin ou double brin.

10. Procédé *in vitro* pour identifier des agents thérapeutiques ou des combinaisons d'agents thérapeutiques efficaces pour induire la différenciation de cellules leucémiques myéloïde, **caractérisé en ce qu'**il comprend les étapes de :

i) mise en culture de cellules issues d'une leucémie myéloïde,
ii) addition d'au moins un composé au milieu de culture de ladite lignée cellulaire,
iii) analyse de l'évolution du niveau d'expression d'au moins un miRNA codé par le précurseur ARN de séquence SEQ ID NO : 13 entre les étapes (i) et (ii),
iv) identification des composés ou des combinaisons de composés entraînant une modification du niveau d'expression dudit miRNA entre les étapes (i) et (ii).

11. Procédé selon la revendication 10, **caractérisé en ce que** l'étape (iii) consiste en l'analyse du niveau d'expression d'au moins un miRNA choisi parmi miR-23a (SEQ ID NO :9), miR-27a (SEQ ID NO :11) et miR-24-2 (SEQ ID NO :12).

12. Procédé selon la revendication 11, **caractérisé en ce que** l'étape (iv) consiste en l'identification des composés ou des combinaisons de composés modulant le niveau d'expression d'au moins un miRNA choisi parmi miR-23a (SEQ ID NO :9), miR-27a (SEQ ID NO :11) et miR-24-2 (SEQ ID NO :12).

13. Procédé selon la revendication 12, **caractérisé en ce que** l'étape (iv) consiste en l'identification des composés ou des combinaisons de composés diminuant le niveau d'expression d'au moins un miRNA choisi parmi miR-23a (SEQ ID NO :9), miR-27a (SEQ ID NO :11) et miR-24-2 (SEQ ID NO :12).

14. Procédé selon l'une quelconque des revendications 10 à 13, **caractérisé en ce que** le composé est un agent thérapeutique pour le traitement du cancer.

15. Procédé selon la revendication 14, **caractérisé en ce que** l'agent thérapeutique est choisi parmi l'AMPc, l'arsenic, les interférons, le TNF, les réxinoïdes, l'acide rétinoïque et les dérivés des rétinoïdes.

16. Procédé selon l'une quelconque des revendications 10 à 15, **caractérisé en ce que** l'étape (iii) d'analyse utilise la technique de northern blot.

17. Procédé selon l'une quelconque des revendications 10 à 16, **caractérisé en ce que** les cellules mises en culture à l'étape (i) sont issues d'une leucémie myéloïde associée à un blocage de la granulopoïèse.

## Claims

1. Use, for producing a drug intended for the treatment of myeloid leukemia, of a nucleic acid molecule having a length of at least 15 nucleotides chosen from the RNA precursor miR23a/24-2 (SEQ ID NO: 13), a derived sequence having at least 80 % identity with such an RNA, a complementary sequence of such an RNA and a derived sequence having at least 80 % identity with such a complementary sequence.

2. Use according to claim 1, **characterized in that** said drug includes a nucleic acid molecule chosen from a complementary sequence of the RNA precursor miR23a/24-2 (SEQ ID NO: 13) and a sequence derived from such a complementary sequence.

3. Use according to any one of claims 1 or 2, **characterized in that** said drug includes a nucleic acid molecule having a sequence chosen from:

   i) the miR23A sequence (SEQ ID NO: 9), a sequence derived from miR23a, the complementary sequence of miR23a, a sequence derived from such a complementary sequence,
   ii) the miR27a sequence (SEQ ID NO: 11), a sequence derived from miR27a, the complementary sequence of miR27a, a sequence derived from such a complementary sequence, and
   iii) the miR24-2 sequence (SEQ ID NO: 12), a sequence derived from miR24-2, the complementary sequence of miR24-2, a sequence derived from such a complementary sequence.

4. Use according to claim 3, **characterized in that** said drug includes a nucleic acid molecule chosen from a complementary sequence of miR23a (SEQ ID NO: 9), miR27a a (SEQ ID NO: 11) and miR24-2 (SEQ ID NO: 12), and the sequences derived from such complementary sequences.

5. Use according to any one of claims 1 to 4, for producing a drug intended for the treatment of myeloid leukemia associated with the inhibition of granulopoiesis.

6. Use according to any one of claims 1 to 5, **characterized in that** the nucleic acid sequences have a length of between 15 and 100 nucleotides.

7. Use according to any one of claims 1 to 6, **characterized in that** the nucleic acid molecules are chosen from DNA and RNA molecules.

8. Use according to any one of claims 1 to 7, **characterized in that** the nucleic acid molecules contain one or more modified nucleotides.

9. Use according to any one of claims 1 to 8, **characterized in that** the nucleic acid molecules are in the form of a single or double strand.

10. *In vitro* process for identifying therapeutic agents or combinations of therapeutic agents effective for inducing myeloid leukemic cell differentiation, **characterized in that** it includes the steps of:

    i) culturing myeloid leukemia cells;
    ii) adding at least one compound to the culture medium of said cell line;
    iii) analyzing the change in the expression level of at least one miRNA coded by the RNA precursor of sequence SEQ ID NO: 13 between steps (i) and (ii);
    iv) identifying compounds or combinations of compounds causing a modification in the expression level of said miRNA between steps (i) and (ii).

11. Process according to claim 10, **characterized in that** step (iii) consists of analyzing the expression level of at least one miRNA chosen from miR-23a (SEQ ID NO: 9), miR-27a (SEQ ID NO: 11) and miR-24-2 (SEQ ID NO: 12).

12. Process according to claim 11, **characterized in that** step (iv) consists of identifying compounds or combinations of compounds modulating the expression level of at least one miRNA chosen from miR-23a (SEQ ID NO: 9), miR-27a (SEQ ID NO: 11) and miR-24-2 (SEQ ID NO: 12).

13. Process according to claim 12, **characterized in that** step (iv) consists of identifying compounds or combinations of compounds diminishing the expression level of at least one miRNA chosen from miR-23a (SEQ ID NO : 9), miR-27a (SEQ ID NO: 11) and miR-24-2 (SEQ ID NO: 12).

**14.** Process according to any one of claims 10 to 13, **characterized in that** the compound is a therapeutic agent for the treatment of cancer.

**15.** Process according to claim 14, **characterized in that** the therapeutic agent is chosen from AMPc, arsenic, interferons, TNF, rexinoids, retinoic acid and retinoid derivatives.

**16.** Process according to any one of claims 10 to 15, **characterized in that** the analysis step (iii) uses the Northern blot technique.

**17.** Process according to any one of claims 10 to 16, **characterized in that** the cells cultured in step (i) are from myeloid leukemia associated with an inhibition of granulopoiesis.

**Patentansprüche**

**1.** Verwendung eines Nukleinsäuremoleküls mit einer Länge von mindestens 15 Nukleotiden zur Herstellung eines zur Behandlung der myeloischen Leukämie bestimmten Arzneimittels, das aus der Vorläufer-RNA miR23a/24-2 (SEQ ID Nr. 13) ausgewählt ist, wobei eine abgeleitete Sequenz eine Identität von mindestens 80% mit einer derartigen RNA aufweist, wobei eine komplementäre Sequenz einer derartigen RNA und eine abgeleitete Sequenz eine Identität von mindestens 80% mit einer derartigen komplementären Sequenz aufweisen.

**2.** Verwendung nach Anspruch 1, **dadurch gekennzeichnet, dass** das Arzneimittel ein Nukleinsäuremolekül umfasst, das aus einer komplementären Sequenz der Vorläufer-RNA miR23a/24-2 (SEQ ID Nr. 13) und einer abgeleiteten Sequenz einer derartigen komplementären Sequenz ausgewählt ist.

**3.** Verwendung nach einem der Ansprüche 1 oder 2, **dadurch gekennzeichnet, dass** das Arzneimittel ein Nukleinsäuremolekül umfasst, das eine Sequenz aufweist, die ausgewählt ist aus:

i) der Sequenz von miR23a (SEQ ID Nr. 9), einer abgeleiteten Sequenz von miR23a, der komplementären Sequenz von miR23a, einer abgeleiteten Sequenz von einer derartigen komplementären Sequenz,
ii) der Sequenz von miR27a (SEQ ID Nr. 11), einer abgeleiteten Sequenz von miR27a, der komplementären Sequenz von miR27a, einer abgeleiteten Sequenz von einer derartigen komplementären Sequenz,
iii) der Sequenz von miR24-2 (SEQ ID Nr. 12), einer abgeleiteten Sequenz von miR24-2, der komplementären Sequenz von miR24-2 und einer abgeleiteten Sequenz von einer derartigen komplementären Sequenz.

**4.** Verwendung nach Anspruch 3, **dadurch gekennzeichnet, dass** das Arzneimittel ein Nukleinsäuremolekül umfasst, das aus einer komplementären Sequenz von miR23a (SEQ ID Nr. 9), von miR27a (SEQ ID Nr. 11) und von miR24-2 (SEQ ID Nr. 12) und den abgeleiteten Sequenzen derartiger komplementärer Sequenzen ausgewählt ist.

**5.** Verwendung nach einem der Ansprüche 1 bis 4 zur Herstellung eines Arzneimittels, das zur Behandlung einer mit einer Blockade der Granulopoiese verbundenen myeloischen Leukämie bestimmt ist.

**6.** Verwendung nach einem der Ansprüche 1 bis 5, **dadurch gekennzeichnet, dass** die Nukleinsäuresequenzen eine Länge zwischen 15 und 100 Nukleotide inklusive haben.

**7.** Verwendung nach einem der Ansprüche 1 bis 6, **dadurch gekennzeichnet, dass** die Nukleinsäuremoleküle aus den DNA- und RNA-Molekülen ausgewählt sind.

**8.** Verwendung nach einem der Ansprüche 1 bis 7, **dadurch gekennzeichnet, dass** die Nukleinsäuremoleküle ein oder mehrere modifizierte Nukleotide enthalten.

**9.** Verwendung nach einem der Ansprüche 1 bis 8, **dadurch gekennzeichnet, dass** die Nukleinsäuremoleküle in Einfach- oder Doppelstrangform sind.

**10.** *in vitro* Verfahren zur Identifikation wirksamer therapeutischer Wirkstoffe oder therapeutischer Wirkstoffkombinationen, um die Differenzierung von myeloischen Leukämiezellen zu induzieren, **dadurch gekennzeichnet, dass** es die Schritte umfasst:

i) Verbringen von myeloischen Leukämiezellen in eine Kultur,
ii) Hinzufügen von mindestens einer Verbindung in das Kulturmedium der besagten Zelllinie,
iii) Analyse der Entwicklung des Expressionsniveaus mindestens einer durch den RNA-Vorläufer der Sequenz SEQ ID Nr. 13 kodierten miRNA zwischen den Schritten (i) und (ii),
iv) Identifizierung der Verbindungen oder Verbindungskombinationen, die eine Modifikation des Expressionsniveaus dieser miRNA zwischen den Schritten (i) und (ii) hervorrufen.

**11.** Verfahren nach Anspruch 10, **dadurch gekennzeichnet, dass** der Schritt (iii) in der Analyse des Expressionsniveaus mindestens einer miRNA besteht, ausgewählt aus miR-23a (SEQ ID Nr. 9), miR-27a (SEQ ID Nr. 11) und miR-24-2 (SEQ ID Nr. 12).

**12.** Verfahren nach Anspruch 11, **dadurch gekennzeichnet, dass** der Schritt (iv) in der Identifikation der Verbindungen oder der Verbindungskombinationen besteht, die das Expressionsniveau mindestens einer miRNA, ausgewählt aus miR-23a (SEQ ID Nr. 9), miR-27a (SEQ ID Nr. 11) und miR-29-2 (SEQ ID Nr. 12), modulieren.

**13.** Verfahren nach Anspruch 12, **dadurch gekennzeichnet, dass** der Schritt (iv) in der Identifikation der Verbindungen oder der Verbindungskombinationen besteht, die das Expressionsniveau mindestens einer miRNA, ausgewählt aus miR-23a (SEQ ID Nr. 9), miR-27a (SEQ ID Nr. 11) und miR-29-2 (SEQ ID Nr. 12), verringern.

**14.** Verfahren nach einem der Ansprüche 10 bis 13, **dadurch gekennzeichnet, dass** die Verbindung ein therapeutischer Wirkstoff zur Behandlung von Krebs ist.

**15.** Verfahren nach Anspruch 14, **dadurch gekennzeichnet, dass** der therapeutische Wirkstoff aus AMPc, dem Arsen, den Interferonen, TNF, den Rexinoiden, der Retinsäure und den Retinoidderivaten ausgewählt ist.

**16.** Verfahren nach einem der Ansprüche 10 bis 15, **dadurch gekennzeichnet, dass** der Analyseschritt (iii) die Northern Blot-Technik verwendet.

**17.** Verfahren nach einem der Ansprüche 10 bis 16, **dadurch gekennzeichnet, dass** die im Schritt (i) in Kultur verbrachten Zellen mit einer Blockade der Granulopoiese verbundene myeloidische Leukämiezellen sind.

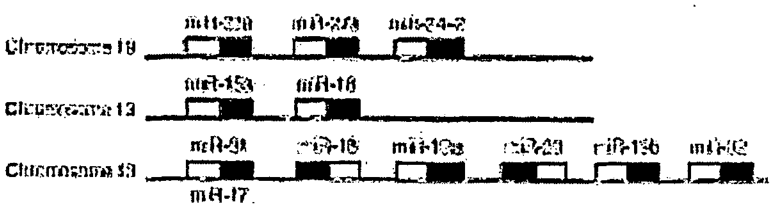

FIG 1

CUCUGCCUCUCCAGUCCUGGGGCUGGAACGGAGGGCACAGCUAGGCUCCA
GCUCCCCGUGUGGUGGCUCCUGCAUAUGAGAAAAGAGCUUCCCUGUGAUC
AAAGGAAGCAUCUGGGGACCUGGAGGGGAGGUGUCCCCAAAUCUCAUUAC
CUCCUUUGCUCUCUCUCUCUUUCUCCCCUCCAGGUGCCAGCCUCUGGCCC
CGCCCGGUGCCCCCCUCACCCCUGUGCCAC***GGCCGGCUGGGGUUCCUGGG***
***GAUGGGAUUUGCUUCCUGUCACAAAUCACAUUGCCAGGGAUUUCCAACCG*** MIR-23A
***ACC***CUGAGCUCUGCCACCGAGGAUGCUGCCCGGGGACGGGGUGGCAGAGA
GGCCCCGAAGCCUGUGCCUGGC***CUGAGGAGCAGGGCUUAGCUGCUUGUGA***
***GCAGGGUCCACACCAAGUCGUGUUCACAGUGGCUAAGUUCCGCCCCCCAG*** MIR-27A
GCCCUCACCUCCUCUGGCCUUGCCGCCUGUCCCCUGCUGCCGCCUGUCUG
CCUGCCAUCCUGCUGCCUGGCCUCCCUGGG***CUCUGCCUCCCGUGCCUACU***
***GAGCUGAAACACAGUUGGUUUGUGUACACUGGCUCAGUUCAGCAGGAACA*** MIR-24-2
***GGG***GUCAAGCCCCCUUGGAGCCUGCAGCCCCUGCCUUCCCUGGGUGGGCU
GAUGCUUGGAGCAGAGAUGAGGACUCAGAAUCAGACCUGUGUCUGGAGGA
GGGAUGUGGUGGGUGGGGUUGGCUGGGCCCAAAUGUGUGCUGCAGGCCCU
GAUCCCCAACUCUGCAACUGGGGACCCCUGCAUGGCCACAGCUCAGGCUG
GGCUGUGGUGCCAGCAUAGAUAGGUGGGUGAGUGGGUGGCCCUUCCAUUA
AAAGGGAAGCCAGCUGUGUCCUUUCCGGGCCUGGAGGCUUGGCCCCUCCU
CUCCCAAGCCUGGCAGGGGCACUGGCCCGGCCCGCACCUUCCUAGCAGCC

FIG 2

UUAGAGUUUGAGGUGUUAAUUCUAAUUAUCUAUUUCAAAUUUAGCAGGAAAAAAGAGAACAU
CACCUUGUAAAACUGAAGAUUGUGACCAGUCAGAAUAAUGUCAAAGUGCUUACAGUGCAGGU
AGUGAUAUGUGCAUCUACUGCAGUGAAGGCACUUGUAGCAUUAUGGUGACAGCUGCCUCGGG
AAGCCAAGUUGGGCUUUAAAGUGCAGGGCCUGCUGAUGUUGAGUGCUUUUUGUUCUAAGGUG
CAUCUAGUGCAGAUAGUGAAGUAGAUUAGCAUCUACUGCCCUAAGUGCUCCUUCUGGCAUAA
GAAGUUAUGUAUUCAUCCAAUAAUUCAAGCCAAGCAAGUAUAUAGGUGUUUUAAUAGUUUUU
GUUUGCAGUCCUCUGUUAGUUUUGCAUAGUUGCACUACAAGAAGAAUGUAGUUGUGCAAAUC
UAUGCAAAACUGAUGGUGGCCUGCUAUUUCCUUCAAAUGAAUGAUUUUUACUAAUUUUGUGU
ACUUUUAUUGUGUCGAUGUAGAAUCUGCCUGGUCUAUCUGAUGUGACAGCUUCUGUAGCACU
AAAGUGCUUAUAGUGCAGGUAGUGUUUAGUUAUCUACUGCAUUAUGAGCACUUAAAGUACUG
CUAGCUGUAGAACUCCAGCUUCGGCCUGUCGCCCAAUCAAACUGUCCUGUUACUGAACACUG
UUCUAUGGUUAGUUUUGCAGGUUUGCAUCCAGCUGUGUGAUAUUCUGCUGUGCAAAUCCAUG
CAAAACUGACUGUGGUAGUGAAAAGUCUGUAGAAAAGUAAGGAAACUCAAACCCCUUUCUA
CACAGGUUGGGAUCGGUUGCAAUGCUGUGUUUCUGUAUGGUAUUGCACUUGUCCCGGCCUGU
UGAGUUUGGUGGGGAUUGUGACCAGAAGAUUUUGAAAAUUAAAUAUUACUGAAGAUUUCGAC
UUCCACUGUUAAAUGUACAAGAUACAUGAAAUAUUAAAGAAAAUGUGUAA

FIG 3

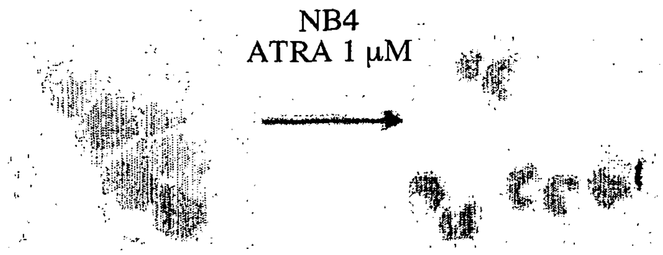


FIG 4

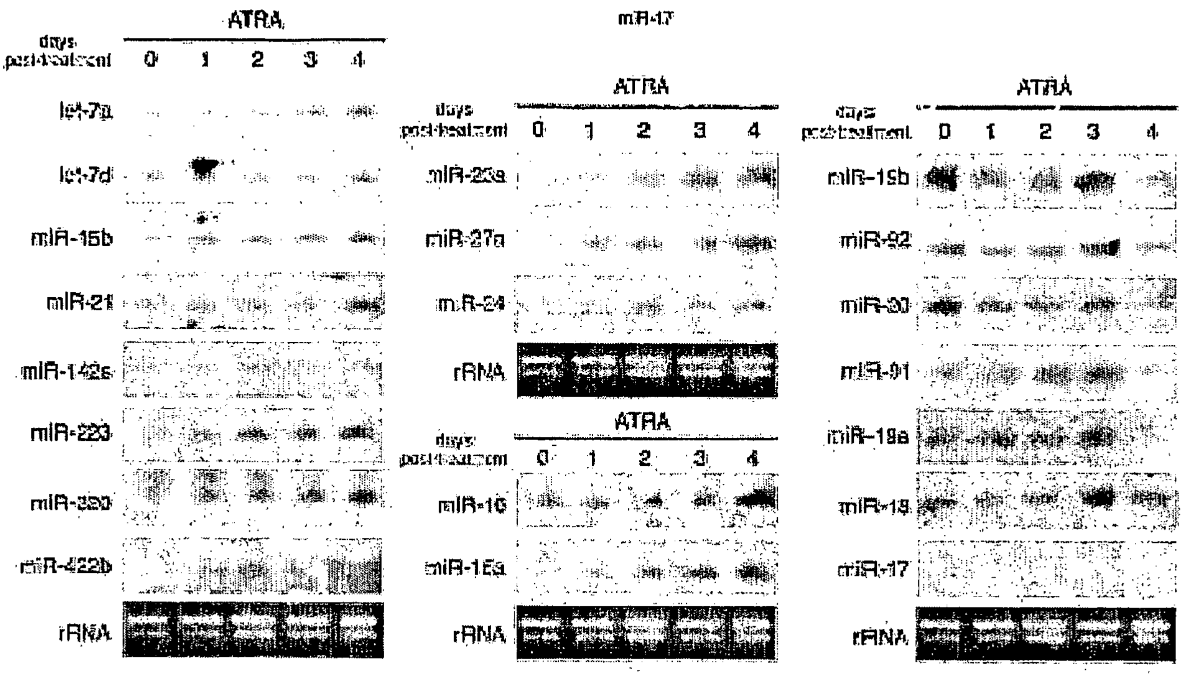
ATRA
days post-treatment
0
1
2
3
4
let-7a
let-7d
miR-15b
miR-21
miR-142s
miR-223
miR-220
miR-422b
rRNA
miR-17
miR-23a
miR-27a
miR-24
miR-16
miR-15a
miR-19b
miR-92
miR-20
miR-91
miR-18a
miR-19


FIG 5

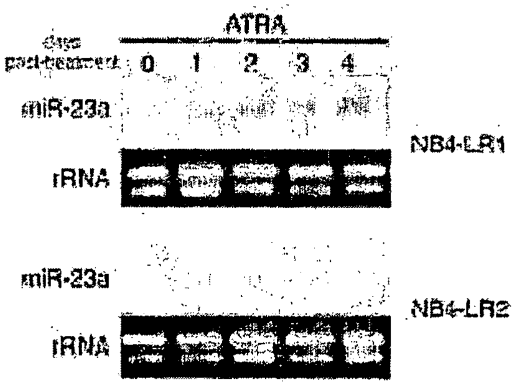
ATRA
days post-treatment
0
1
2
3
4
miR-23a
rRNA
NB4-LR1
NB4-LR2


FIG 6

GAUGAAGAUGUCUUUUGAAAGGUGUACUGCAAGGAACAAAAUGUUUGUAAAUUCUCCUUUUA
CCAAGGUAAAGAUCAAAUUUUAUAAAUUUACUUGUUUGUUUAUACAAGGAAAAAUAACUUCA
UAUAUUGAAUAUAUUCAAAAGUUUAAGCAUUUAGUUGUAUUGCCCUGUUAAGUUGGCAUAGC
AAAUAAAUGCUUUUCUUUUCCUCAUUUUAUUCUUUGUGUUUCCUAACCUAUAGCACUGUGCU
GGGCACAGAAUGGACUUCAGUUAAGUUUUUGAUGUAGAAAUGUUUUAUUAUUCUACUUAAAA
UCUCCUUAAAAAUAAUUAUGCAUAUUACAUCAAUGUUAUAAUGUUUAAACAUAGAUUUUUUU
ACAUGCAUUCUUUUUUUCCUGAAAGAAAAUAUUUUUUAUAUUCUUUAGGCGCGAAUGUGUGU
UUAAAAAAAAUAAAACCUUGGAGUAAAGUAGCAGCACAUAAUGGUUUGUGGAUUUUGAAAAG
GUGCAGGCCAUAUUGUGCUGCCUCAAAAAUACAAGGAUCUGAUCUUCUGAAGAAAAUAUAUU
UCUUUUUAUUCAUAGCUCUUAUGAUAGCAAUGUCAGCAGUGCCUUAGCAGCACGUAAAUAUU
GGCGUUAAGAUUCUAAAAUUAUCUCCAGUAUUAACUGUGCUGCUGAAGUAAGGUUGACCAUA
CUCUACAGUUGUGUUUUAAUGUAUAUUAAUGUUACUAAUGUGUUUCAGUUUUAUUGAUAGU
CUUUUCAGUAUUAUUGAUAAUCUUGUUAUUUUUAGUAUGAUUCUGUAAAAAUGAAUUAAUAC
UAAUUUUUCAGAUGUAUCAUCUCUUAAAAUACUGUAAUUGCAAUUUAAUAAUUGUAUUGAAU
GCCAUCAAGUUUUUUUAAAAAGCUUAUGCAGCAUUAGAGGAAUUUAUUUUAAUGCACAUUUA
UAUUCAACAUAGACAUUAAUUCAGAUUUUUACUUGGGAUAAAACAAAUUCUAGUUUUCCCUU
UGUUUUGAAAUUACUUUUAAAAUAUGUCUUUACAGAUAAAUAUAAAAUAUAUUAAGCAUUUU
GAACAGAGCUUAGAAGACAAUAUUUAGUACUGUUUCUGAAUAUUUCUUUAUAUCUGAAGGGG
AAAAGCCAUC

FIG 7

TTAGAGTTTGAGGTGTTAATTCTAATTATCTATTTCAAATTTAGCAGGAAAAAAGAGAACAT
CACCTTGTAAAACTGAAGATTGTGACCAGTCAGAATAATGTCAAAGTGCTTACAGTGCAGGT
AGTGATATGTGCATCTACTGCAGTGAAGGCACTTGTAGCATTATGGTGACAGCTGCCTCGGG
AAGCCAAGTTGGGCTTTAAAGTGCAGGGCCTGCTGATGTTGAGTGCTTTTTGTTCTAAGGTG
CATCTAGTGCAGATAGTGAAGTAGATTAGCATCTACTGCCCTAAGTGCTCCTTCTGGCATAA
GAAGTTATGTATTCATCCAATAATTCAAGCCAAGCAAGTATATAGGTGTTTTAATAGTTTTT
GTTTGCAGTCCTCTGTTAGTTTTGCATAGTTGCACTACAAGAAGAATGTAGTTGTGCAAATC
TATGCAAAACTGATGGTGGCCTGCTATTTCCTTCAAATGAATGATTTTTACTAATTTTGTGT
ACTTTTATTGTGTCGATGTAGAATCTGCCTGGTCTATCTGATGTGACAGCTTCTGTAGCACT
AAAGTGCTTATAGTGCAGGTAGTGTTTAGTTATCTACTGCATTATGAGCACTTAAAGTACTG
CTAGCTGTAGAACTCCAGCTTCGGCCTGTCGCCCAATCAAACTGTCCTGTTACTGAACACTG
TTCTATGGTTAGTTTTGCAGGTTTGCATCCAGCTGTGTGATATTCTGCTGTGCAAATCCATG
CAAAACTGACTGTGGTAGTGAAAAGTCTGTAGAAAAGTAAGGGAAACTCAAACCCCTTTCTA
CACAGGTTGGGATCGGTTGCAATGCTGTGTTTCTGTATGGTATTGCACTTGTCCCGGCCTGT
TGAGTTTGGTGGGGATTGTGACCAGAAGATTTTGAAAATTAAATATTACTGAAGATTTCGAC
TTCCACTGTTAAATGTACAAGATACATGAAATATTAAAGAAAATGTGTAA

FIG 8

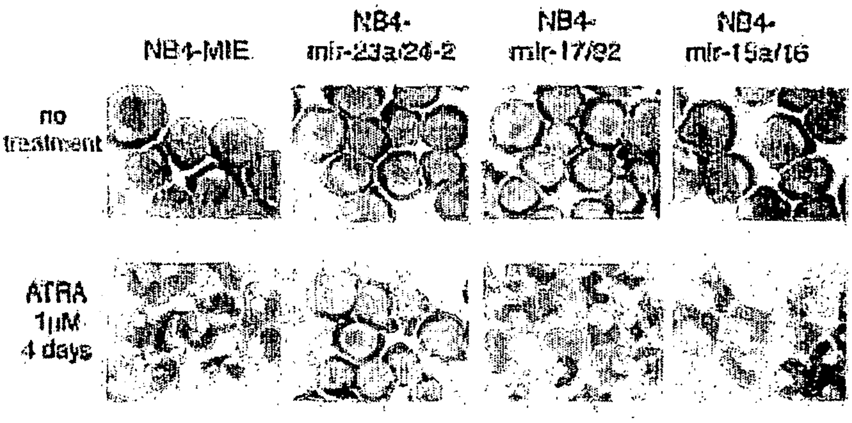
NB4-MIE
NB4-
mir-23a/24-2
NB4-
mir-17/92
NB4-
mir-15a/16
no
treatment
ATRA
1μM
4 days

FIG 9

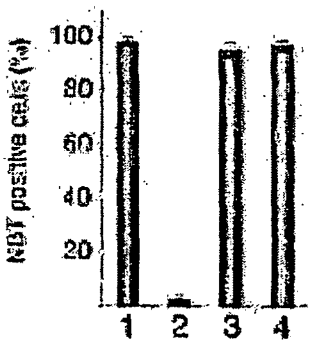
NBT positive cells (%)
100
80
60
40
20
1
2
3
4

FIG 10

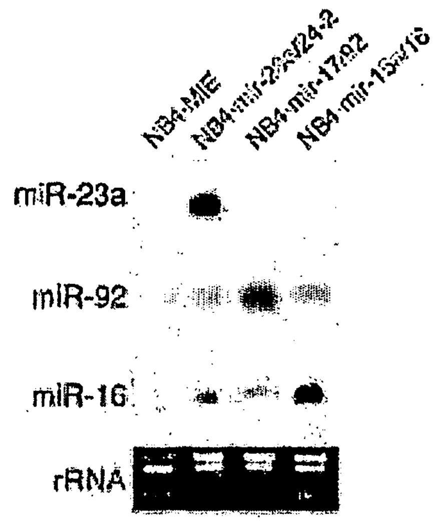
NB4-MIE
NB4-mir-23a/24-2
NB4-mir-17/92
miR-23a
miR-92
miR-16
rRNA

FIG 11

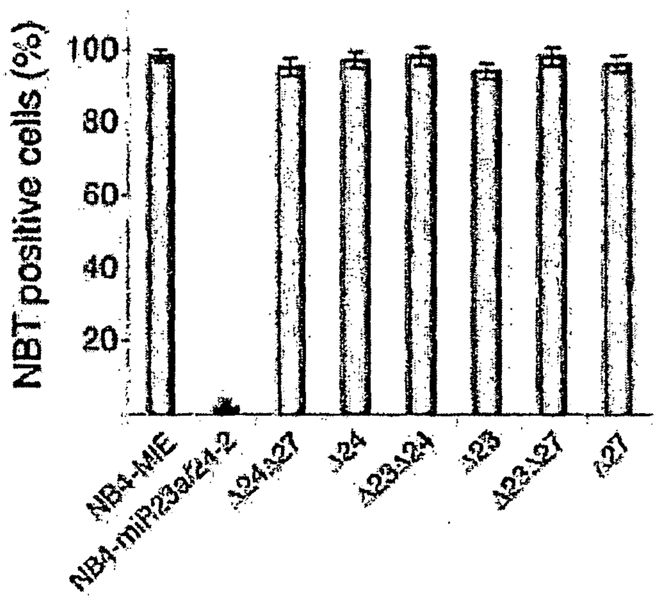
NBT positive cells (%)
100
80
60
40
20
NB4-MIE
NB4-miR23a/24-2
Δ24Δ27
Δ24
Δ23Δ24
Δ23
Δ23Δ27
Δ27

FIG 12

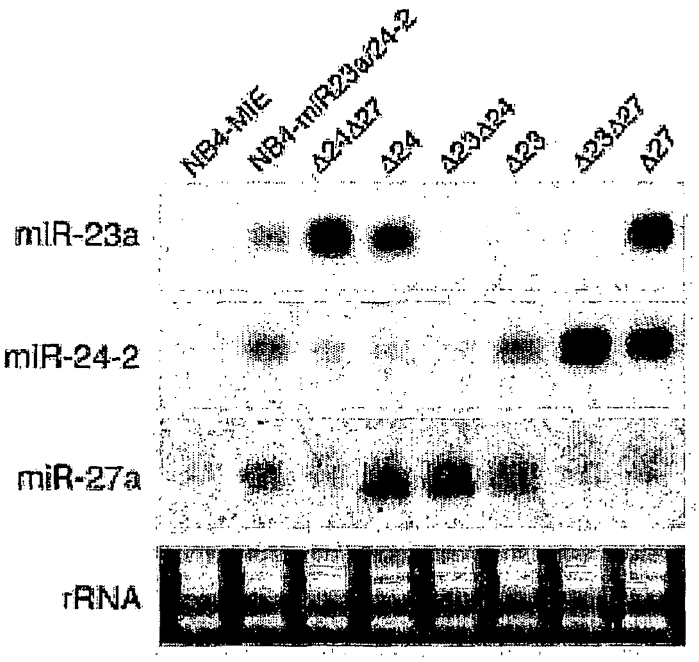


FIG 13

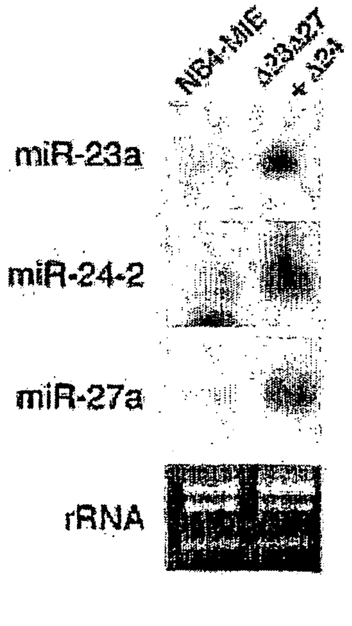


FIG 15

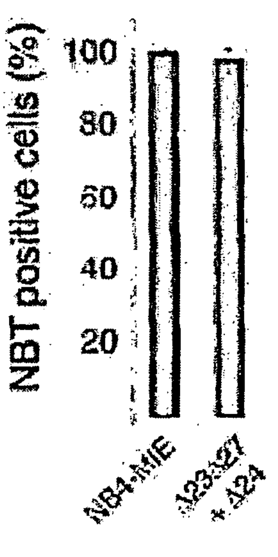


FIG 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03029459 A
- FR 0411725


### Littérature non-brevet citée dans la description


- **YEKTA et al.** *Science,* 2004, vol. 304, 594-596
- **MANSFIELD.** *Nat Genet.,* vol. 36 (10), 1079-83
- **BARTEL D.P.** *Cell,* 2004, vol. 116, 281-297
- **CHEN et al.** *Science,* vol. 303 (5654), 83-6
- **BERSTEIN et al.** *Nat. Genet.,* 2003, vol. 35, 215-7
- **TAKAMIZAWA et al.** *Cancer Res.,* 2004, vol. 64, 3753-3756
- **DE THE ; CHELBI-ALIX.** *oncogene,* 2001, vol. 20, 7136-9
- **BENOIT et al.** *Oncogene,* 2001, vol. 20, 7161-7177
- **LANOTTE et al.** *Blood,* 1991, vol. 77, 1080-1086
- **LLAVE et al.** *Plant Cell,* 2002, vol. 14, 1605-1619
- **GRAHAM ; VAN DER EB.** *Virol.,* 1973, vol. 52, 456
- **MCCUTHAN ; PAGANO.** *J. Natl. Cancer Inst.,* 1968, vol. 41, 351
- **CHU et al.** *Nucl. Acids Res.,* vol. 15, 1311
- **FRALEY et al.** *J. Biol. Chem.,* 1980, vol. 255, 10431
- **CAPECCHI et al.** *Cell,* 1980, vol. 22, 479
- **FELGNER.** *Proc. Natl. Acad. Sci. USA,* 1988, vol. 84, 7413
- **RUCHAUD et al.** *Proc. Natl. Acad. Sci.,* 1994, vol. 91, 8428-8432
- **LEE et al.** *Embo J.,* 2002, vol. 21, 4663-4670
- **CHANGCHUN et al.** *Blood,* 1999, vol. 94 (2), 793-802
- **PEAR et al.** *Proc. Natl. Acad. Sci. USA,* 1993, vol. 90, 8392-8396
- **LAVAU et al.** *EMBO J,* 1997, vol. 16, 4226-4237
- **MEISTER et al.** *RNA,* vol. 10 (3), 544-502004

SEQUENCE LISTING

<110> Centre National de la Recherche Scientifique (CNRS)
VOINNET, Olivier
LECELLIER, Charles-Henri
SAUMET, Anne
LANOTTE, Michel

<120> Utilisation des miRNAs dans le traitement de la leucémie myéloïde

<130> 36661/PCT

<150> FR 04/11725
<151> 2004-11-03

<160> 23

<170> PatentIn version 3.3

<210> 1
<211> 24
<212> RNA
<213> Homo sapiens

<400> 1
caaagugcuu acagugcagg uagu 24

<210> 2
<211> 22
<212> RNA
<213> Homo sapiens

<400> 2
uaaggugcau cuagugcaga ua 22

<210> 3
<211> 23
<212> RNA
<213> Homo sapiens

<400> 3
ugugcaaauc uaugcaaaac uga 23

<210> 4
<211> 23
<212> RNA
<213> Homo sapiens

<400> 4
ugugcaaauc caugcaaaac uga 23

<210> 5
<211> 22
<212> RNA
<213> Homo sapiens

<400> 5
uaaagugcuu auagugcagg ua 22

<210> 6
<211> 22
<212> RNA
<213> Homo sapiens

<400> 6
uauugcacuu gucccggccu gu 22

<210> 7
<211> 980
<212> RNA
<213> Homo sapiens

<400> 7

uuagaguuug agguguuaau ucuaauuauc uauuucaaau uuagcaggaa aaaagagaac 60

aucaccuugu aaaacugaag auugugacca gucagaauaa ugucaaagug cuuacagugc 120

agguagugau augugcaucu acugcaguga aggcacuugu agcauuaugg ugacagcugc 180

cucgggaagc caaguugggc uuuaaagugc agggccugcu gauguugagu gcuuuuuguu 240

cuaaggugca ucuagugcag auagugaagu agauuagcau cuacugcccu aagugcuccu 300

ucuggcauaa gaaguuaugu auucauccaa uaauucaagc caagcaagua uauagguguu 360

uuaauaguuu uuguuugcag uccucuguua guuuugcaua guugcacuac aagaagaaug 420

uaguugugca aaucuaugca aaacugaugg uggccugcua uuuccuucaa augaaugauu 480

uuuacuaauu uuguguacuu uuauuguguc gauguagaau cugccugguc uaucugaugu 540

gacagcuucu guagcacuaa agugcuuaua gugcagguag uguuuaguua ucuacugcau 600

uaugagcacu uaaaguacug cuagcuguag aacuccagcu ucggccuguc gcccaaucaa 660

acuguccugu uacugaacac uguucuaugg uuaguuuugc agguuugcau ccagcugugu 720

gauauucugc ugugcaaauc caugcaaaac ugacuguggu agugaaaagu cuguagaaaa 780

guaagggaaa cucaaacccc uuucuacaca gguugggauc gguugcaaug cuguguuucu 840

guaugguauu gcacuugucc cggccuguug aguuuggugg ggauugugac cagaagauuu 900

ugaaaauuaa auauuacuga agauuucgac uuccacuguu aaauguacaa gauacaugaa 960

auauuaaaga aaauguguaa 980

<210> 8
<211> 20
<212> RNA
<213> Homo sapiens

<400> 8
acugcaguga aggcacuugu 20

<210> 9
<211> 22
<212> RNA
<213> Homo sapiens

<400> 9
aucacauugc cagggauuuc ca 22

<210> 10

<211> 980
<212>. DNA
<213> Homo sapiens

<400> 10

ttagagtttg aggtgttaat tctaattatc tatttcaaat ttagcaggaa aaaagagaac 60
atcaccttgt aaaactgaag attgtgacca gtcagaataa tgtcaaagtg cttacagtgc 120
aggtagtgat atgtgcatct actgcagtga aggcacttgt agcattatgg tgacagctgc 180
ctcgggaagc caagttgggc tttaaagtgc agggcctgct gatgttgagt gctttttgtt 240
ctaaggtgca tctagtgcag atagtgaagt agattagcat ctactgccct aagtgctcct 300
tctggcataa gaagttatgt attcatccaa taattcaagc caagcaagta tataggtgtt 360
ttaatagttt ttgtttgcag tcctctgtta gttttgcata gttgcactac aagaagaatg 420
tagttgtgca aatctatgca aaactgatgg tggcctgcta tttccttcaa atgaatgatt 480
tttactaatt ttgtgtactt ttattgtgtc gatgtagaat ctgcctggtc tatctgatgt 540
gacagcttct gtagcactaa agtgcttata gtgcaggtag tgtttagtta tctactgcat 600
tatgagcact taaagtactg ctagctgtag aactccagct tcggcctgtc gcccaatcaa 660
actgtcctgt tactgaacac tgttctatgg ttagttttgc aggtttgcat ccagctgtgt 720
gatattctgc tgtgcaaatc catgcaaaac tgactgtggt agtgaaaagt ctgtagaaaa 780
gtaagggaaa ctcaaacccc tttctacaca ggttgggatc ggttgcaatg ctgtgtttct 840
gtatggtatt gcacttgtcc cggcctgttg agtttggtgg ggattgtgac cagaagattt 900
tgaaaattaa atattactga agatttcgac ttccactgtt aaatgtacaa gatacatgaa 960
atattaaaga aaatgtgtaa 980

<210> 11
<211> 21
<212> RNA
<213> Homo sapiens

<400> 11
uucacagugg cuaaguuccg c 21

<210> 12
<211> 22
<212> RNA
<213> Homo sapiens

<400> 12
uggcucaguu cagcaggaac ag 22

<210> 13
<211> 950

<212> RNA
<213> Homo sapiens

<400> 13

cucugccucu ccaguccugg ggcuggaacg gagggcacag cuaggcucca gcuccccgug 60
ugguggcucc ugcauaugag aaaagagcuu cccugugauc aaaggaagca ucuggggacc 120
uggaggggag guguccccaa aucucauuac cuccuuugcu cucucucucu uucuccccuc 180
caggugccag ccucuggccc cgcccggugc cccccucacc ccugugccac ggccggcugg 240
gguuccuggg gaugggauuu gcuuccuguc acaaaucaca uugccaggga uuuccaaccg 300
acccugagcu cugccaccga ggaugcugcc cggggacggg guggcagaga ggccccgaag 360
ccugugccug gccugaggag cagggcuuag cugcuuguga gcaggcucca caccaagucg 420
uguucacagu ggcuaaguuc cgccccccag gcccucaccu ccucuggccu ugccgccugu 480
ccccugcugc cgccugucug ccugccaucc ugcugccugg ccucccuggg cucugccucc 540
cgugccuacu gagcugaaac acaguugguu uguguacacu ggcucaguuc agcaggaaca 600
ggggucaagc ccccuuggag ccugcagccc cugccuuccc uggguggggcu gaugcuugga 660
gcagagauga ggacucagaa ucagaccugu gucuggagga gggauguggu ggguggguu 720
ggcugggccc aaaugugugc ugcaggcccu gaucccccaac ucugcaacug gggaccccug 780
cauggccaca gcucaggcug ggcuguggug ccagcauaga uaggugggug agugggugggc 840
ccuuccauua aaagggaagc cagcuguguc cuuuccgggc cuggaggcuu ggccccuccu 900
cucccaagcc uggcaggggc acuggcccgg cccgcaccuu ccuagcagcc 950

<210> 14
<211> 22
<212> RNA
<213> Homo sapiens

<400> 14
uagcagcaca uaaugguuug ug 22

<210> 15
<211> 22
<212> RNA
<213> Homo sapiens

<400> 15
uagcagcacg uaaauauugg cg 22

<210> 16
<211> 1126
<212> RNA
<213> Homo sapiens

<400> 16

gaugaagaug ucuuuugaaa gguguacugc aaggaacaaa auguuuguaa auucuccuuu 60
uaccaaggua aagaucaaau uuuauaaauu uacuuguuug uuuauacaag gaaaaauaac 120
uucauauauu gaauauauuc aaaaguuuaa gcauuuaguu guauugcccu guuaaguugg 180
cauagcaaau aaaugcuuuu cuuuuccuca uuuuauucuu uguguuuccu aaccuauagc 240
acugugcugg gcacagaaug gacuucaguu aaguuuuuga uguagaaaug uuuuauuauu 300
cuacuuaaaa ucuccuuaaa aauaauuaug cauauuacau caauguuaua auguuuaaac 360
auagauuuuu uuacaugcau ucuuuuuuuc cugaaagaaa auauuuuuua uauucuuuag 420
gcgcgaaugu guguuuaaaa aaaauaaaac cuuggaguaa aguagcagca cauaaugguu 480
uguggauuuu gaaaaggugc aggccauauu gugcugccuc aaaaauacaa ggaucugauc 540
uucugaagaa aauauauuuc uuuuuauuca uagcucuuau gauagcaaug ucagcagugc 600
cuuagcagca cguaaauauu ggcguuaaga uucuaaaauu aucuccagua uuaacugugc 660
ugcugaagua agguugacca uacucuacag uuguguuuua auguauauua auguuacuaa 720
uguguuuuca guuuuauuga uagucuuuuc aguauuauug auaaucuugu uauuuuuagu 780
augauucugu aaaaaugaau uaauacuaau uuuucagaug uaucaucucu uaaaauacug 840
uaauugcaau uuaauaauug uauugaaugc caucaaguuu uuuuaaaaag cuuaugcagc 900
auuagaggaa uuuauuuuaa ugcacauuua uauucaacau agacauuaau ucagauuuuu 960
acuugggaua aaacaaauuc uaguuuuccc uuuguuuuga aauuacuuuu aaaauauguc 1020
uuuacagaua aauauaaaau auauuaagca uuuugaacag agcuuagaag acaauauuua 1080
guacuguuuc ugaauauuuc uuuauaucug aaggggaaaa gccauc 1126

<210> 17
<211> 22

<212> RNA
<213> Homo sapiens

<400> 17
ugagguagua gguuguauag uu 22

<210> 18
<211> 21
<212> RNA
<213> Homo sapiens

<400> 18
agagguagua gguugcauag u 21

<210> 19

<211> 22
<212> RNA
<213> Homo sapiens

<400> 19
uagcagcaca ucaugguuua ca 22

<210> 20
<211> 20
<212> RNA
<213> Homo sapiens

<400> 20
cauaaaguag aaagcacuac 20

<210> 21
<211> 21
<212> RNA
<213> Homo sapiens

<400> 21
ugucaguuug ucaaauaccc c 21

<210> 22
<211> 23
<212> RNA
<213> Homo sapiens

<400> 22
aaaagcuggg uugagagggc gaa 23

<210> 23
<211> 22
<212> RNA
<213> Homo sapiens

<400> 23
cuggacuugg agucagaagg cc 22